# EUROPEAN PATENT SPECIFICATION

(11) **EP 4 530 357 B1**
(45) Date of publication and mention of the grant of the patent: **04.11.2026**
(21) Application number: 23200244.4
(22) Date of filing: 27.09.2023
(51) Int. Cl.: C12Q 1/682

(54) **ENHANCED NUCLEIC-ACID DETECTION USING TRUNCATED TARGET NUCLEIC ACIDS**
VERBESSERTER NUKLEINSÄURENACHWEIS MIT VERKÜRZTEN ZIELNUKLEINSÄUREN
DÉTECTION AMÉLIORÉE D'ACIDES NUCLÉIQUES À L'AIDE D'ACIDES NUCLÉIQUES CIBLES TRONQUÉS

(43) Date of publication of application: 02.04.2025
(73) Proprietor: Helmholtz-Zentrum für Infektionsforschung GmbH, 38124 Braunschweig (DE)
(72) Inventor: BEISEL, Chase, 38124 Braunschweig (DE); JIAO, Chunlei, 38124 Braunschweig (DE)
(74) Representative: Krauss, Jan

(56) References cited:
- WO-A1-2019/104058
- WANG ZUPENG ET AL: "Cas12c-DETECTOR: A specific and sensitive Cas12c-based DNA detection platform", INTERNATIONAL JOURNAL OF BIOLOGICAL MACROMOLECULES, ELSEVIER BV, NL, vol. 193, 26 October 2021 (2021-10-26), pages 441 - 449, XP086865827, ISSN: 0141-8130, [retrieved on 20211026], DOI: 10.1016/J.IJBIOMAC.2021.10.167
- JANICE S. CHEN ET AL: "CRISPR-Cas12a target binding unleashes indiscriminate single-stranded DNase activity", SCIENCE, vol. 360, no. 6387, 27 April 2018 (2018-04-27), US, pages 436 - 439, XP055615609, ISSN: 0036-8075, DOI: 10.1126/science.aar6245
- CHEN JANICE S. ET AL: "Supplementary Materials for CRISPR-Cas12a target binding unleashes indiscriminate single-stranded DNase activity", SCIENCE, vol. 360, no. 6387, 27 April 2018 (2018-04-27), US, pages 436 - 439, XP093119764, ISSN: 0036-8075, Retrieved from the Internet <URL:https://www.science.org/doi/suppl/10.1126/science.aar6245/suppl_file/aar6245_chen_sm.pdf> DOI: 10.1126/science.aar6245
- RANANAWARE SANTOSH R. ET AL: "Programmable RNA detection with CRISPR-Cas12a", BIORXIV, 30 January 2023 (2023-01-30), pages 1 - 36, XP093032671, Retrieved from the Internet <URL:https://www.biorxiv.org/content/10.1101/2023.01.29.525716v1> [retrieved on 20230317], DOI: 10.1101/2023.01.29.525716
- JIAO CHUNLEI ET AL: "Noncanonical crRNAs derived from host transcripts enable multiplexable RNA detection by Cas9", SCIENCE, vol. 372, no. 6545, 28 May 2021 (2021-05-28), US, pages 941 - 948, XP093119593, ISSN: 0036-8075, Retrieved from the Internet <URL:https://www.ncbi.nlm.nih.gov/pmc/articles/PMC8224270/pdf/372_941.pdf> DOI: 10.1126/science.abe7106
- SMITH CHRISTOPHER W. ET AL: "Recognition of DNA Target Formulations by CRISPR-Cas12a Using a dsDNA Reporter", ACS SYNTHETIC BIOLOGY, vol. 10, no. 7, 16 July 2021 (2021-07-16), Washington DC ,USA, pages 1785 - 1791, XP093019164, ISSN: 2161-5063, Retrieved from the Internet <URL:https://www.ncbi.nlm.nih.gov/pmc/articles/PMC9190466/pdf/nihms-1812802.pdf> DOI: 10.1021/acssynbio.1c00204
- MONTAGUD-MARTÍNEZ ROSER ET AL: "CRISPR-Cas9 trans-cleavage is hindered by a flanked R-loop, an elongated spacer, and an inactive HNH domain", NATURE COMMUNICATIONS, vol. 17, no. 1, 24 January 2026 (2026-01-24), UK, XP093402316, ISSN: 2041-1723, DOI: 10.1038/s41467-026-68789-3

## Description

The present invention relates to a CRISPR diagnostic method for detecting at least one specific dsDNA target nucleic acid, comprising at least one suitably shortened dsDNA target nucleic acid molecule. Preferably, the binding of the at least one dsDNA target nucleic acid by a Cas nuclease enzyme is dependent from an RNA molecule to be detected, preferably an mRNA molecule to be detected. The method may be performed in the absence of gRNA molecules. Further provided are novel dsDNA target nucleic acid molecules for the method and their uses, as well as a kit for performing the method according to the invention.

### Background of the invention

CRISPR (Clustered Regularly Interspaced Short Palindromic Repeats)-based diagnostics are gaining popularity as fast, affordable, and portable alternatives to traditional PCR-based assays (1). The mostly widely adopted formats have involved the single-effector CRISPR-associated (Cas) nucleases Cas12a and Cas13 because of their respective ability to collaterally cleave non-specific single-stranded (ss)DNA or ssRNA reporters upon guide RNA (gRNA)-directed recognition of a target double-stranded (ds)DNA or RNA sequence (2-4).

With the discovery of a wide assortment of Cas12 orthologs now grouped into over a dozen sub-types (V-A through V-N) (5-8), many of these nucleases have been co-opted for nucleic-acid detection (2, 9-12). A few of these nucleases were shown to recognize RNA, either as their sole nucleic-acid targets (Cas12a2, Cas12g) with the requirement for an adjacent protospacerflanking sequence (12-15) or as one of numerous nucleic-acid targets (Cas12f1) (16). However, the vast majority of these nucleases require dsDNA targets to elicit collateral cleavage activity. In turn, dsDNA detection technologies based on Cas12 nucleases have been a major focus for numerous applications ranging from disease diagnosis to food quality control and the detection of environmental pollutants 1, 17, 18).

Despite the prominence of dsDNA-targeting Cas12 nucleases for nucleic-acid detection, they come with two notable restrictions: i) limitation to dsDNA targets, and ii) a strict requirement for a flanking protospacer-adjacent motif (PAM). Tackling the first restriction, multiple studies have recently reported PAM-free detection of dsDNA using generated ssDNA targets (19-21), generated dsDNA targets with a defined single-stranded overhang (22), or dsDNA targets flanked by PAMs introduced as part of DNA amplification (23-26). However, none allowed the direct detection of RNA.

Tackling both restrictions, one study reported PAM-free RNA detection with Cas12a by providing a dsDNA encoding a PAM and the PAM-proximal end of the gRNA guide, with a ssDNA PAM-distal end of the gRNA guide available for RNA recognition (27). While an innovative use of splitting the target to detect RNA, only the PAM-distal end of the guide (i.e., the portion less sensitive to mismatches (2)) is available for sequence-specific recognition. Therefore, Cas12 nucleases remain to be fully leveraged for direct, PAM-free detection of RNA.

One distinct yet quite unexplored strategy involves tracrRNAs (trans-activating CRISPR RNAs) (28). tracrRNAs play an essential role in gRNA biogenesis as part of type II CRISPR-Cas systems, the source of Cas9 nucleases, and most type V CRISPR-Cas systems, the source of Cas12 nucleases (5). In all cases, the tracrRNA hybridizes to the conserved repeat region in CRISPR RNAs (crRNAs) associated with CRISPR-Cas systems (28). The formed RNA duplex then undergoes processing typically by the endoribonuclease RNase III and complexes with the cognate Cas nuclease. The bound RNA duplex then functions as a gRNA that directs the nuclease to bind and cleave target dsDNA.

Baisong, T., et al. (in: The Versatile Type V CRISPR Effectors and Their Application Prospects, Frontiers in Cell and Developmental Biology, Vol. 8, 2021, p. 1835, DOI: 10.3389/fcell.2020.622103) disclose that the class 2 clustered regularly interspaced short palindromic repeats (CRISPR)-Cas systems, characterized by a single effector protein, can be further subdivided into types II, V, and VI. The application of the type II CRISPR effector protein Cas9 as a sequence-specific nuclease in gene editing has revolutionized the field of DNA manipulation. Similarly, Cas13 as the effector protein of type VI provides a convenient tool for RNA manipulation. Additionally, the type V CRISPR-Cas system is another valuable resource with many subtypes and diverse functions. In their review, they summarize all the subtypes of the type V family that have been identified so far. According to the functions currently displayed by the type V family, they attempt to introduce the functional principle, current application status, and development prospects in biotechnology for all major members.

WO 2022/253903A1 relates to methods for RNA-directed cleaving of a nucleic acid molecule selected from dsDNA, ssDNA, and RNA based on a complex comprising a CasΩ nuclease and at least one pre-selected guide RNA designed for binding to at least one target RNA. Further provided is the complex of the present invention bound to a target RNA molecule, as well as respective systems for cleaving of a nucleic acid molecule, and diagnostic and therapeutic uses thereof.

Zhang W, et al. (in: PAM-independent ultra-specific activation of CRISPR-Cas12a via sticky-end dsDNA. Nucleic Acids Res. 2022 Dec 9;50(22):12674-12688. doi: 10.1093/nar/gkac1144. PMID: 36484104) describe a new targeting substrate for LbaCas12a (Lachnospiraceae bacterium Cas12a), namely double-stranded DNA (dsDNA) with a sticky-end region (PAM-SE+ dsDNA). The results indicated that the recognition efficiency gradually increased with the enlargement of the initiation region, up to 5 nt. CRISPR-Cas12a had special enzymatic properties for this substrate DNA, including the ability to recognize and cleave it without needing a protospacer adjacent motif (PAM) sequence and a high sensitivity to single-base mismatches in that substrate. The truncated end lies at the PAM-proximal end of the dsDNA target, circumventing the need for a PAM. However, the dsDNA target must still nick both strands of the downstream dsDNA before the cleavage of collateral substrates can be initiated.

Rananaware Santosh R. et al. (in: Programmable RNA detection with CRISPR-Cas12a, bioiv, 30 January 2023, pages 1-36, DOI: 10.1101/2023.01.29.525716) describe a method named Split Activators for Highly Accessible RNA Analysis or 'SAHARA' to detect RNA sequences at the PAM-distal region of the cNA by merely supplying a short ssDNA or a PAM containing dsDNA to the seed region. Amplification-free detection of picomolar concentrations of miRNA-155 and hepatitis C virus RNA is described.

Jiao CHUNLEI et al. (in: Noncanonical cNAs derived from host transcripts enable multiplexable RNA detection by Cas9, SCIENCE, vol. 372, no. 6545, 28 May 2021, pages 941 -948, ISSN: 0036-8075, DOI: 10.1 126/science.abe7106) describe engineering of reprogrammed tracNAs that link the presence of any RNA of interest to DNA targeting with different C359 orthologs. This capability became the basis for a multiplexable diagnostic platform termed LEOPARD (leveraging engineered tracNAs and on-target DNAs for parallel RNA detection). The LEOPARD platform achieves multiplex RNA detection in a single reaction by monitoring the sequence-specific cleavage of parallel DNA targets by Cas9 in a gel-based readout.

It is an object of the present invention to provide an improved CRISPR-derived dsDNA-targeting assay for nucleic-acid detection that, at least in part, overcomes the above restrictions and allows for the direct detection of RNA molecules to be detected. Other objects and advantages of the present invention will become apparent to the person of skill in the art upon studying the present disclosure further.

According to a first aspect thereof, the above object has been solved according to the present invention by a CRISPR diagnostic method for detecting at least one specific dsDNA target nucleic acid, comprising the steps of
targeting or binding the at least one dsDNA target nucleic acid by a Cas nuclease enzyme, wherein the Cas nuclease enzyme is selected from a Cas enzyme binding to dsDNA nucleic acid molecules, such as, for example, Cas9 or Cas12, in particular Cas12a, Cas12b, Cas12c, Cas12d, Cas12e, Cas12f1, Cas12g, Cas12i, and Cas12l, and subsequently cleaving of at least one marker nucleic acid by collateral nuclease activity of the Cas nuclease enzyme, comprising providing the at least one dsDNA target nucleic acid comprising
a target strand (TS) sequence comprising a Cas nuclease cleavage site and a non-target strand (NTS) sequence forming a TS:NTS duplex,
at least one protospacer adjacent motif (PAM) sequence directly or adjacently located upstream or downstream from said TS sequence, and
a sequence extending upstream and/or downstream from the PAM and/or TS:NTS duplex,
wherein before the step of binding the dsDNA target nucleic acid molecule by the Cas nuclease enzyme, the sequence extending from the TS:NTS duplex is shortened to 30 base pairs or less, and is preferably eliminated and if the downstream extended sequence is eliminated, the TS sequence is truncated between 0 to 8, preferably between 2 to 4, nucleotide bases and/or the NTS sequence is truncated between 0 to 10, preferably between 6 to 8, nucleotide bases.

Preferred is the method according to the present invention, wherein the length of the shortened at least one dsDNA target nucleic acid is between about 120 and about 24 bp, preferably between about 100 and about 30 bp, more preferably at between about 60 and about 35 bp.

In a first aspect thereof, the present invention relates to the reprogramming or modification of tracrRNAs associated with diverse type V CRISPR-Cas systems to address two general challenges with traditional Cas12-based detection technologies: direct RNA detection and PAM requirements. Rptrs allow dsDNA-targeting Cas12 nucleases to directly detect RNA by binding and converting RNAs to be detected into guide RNAs for dsDNA targeting. The detected substrates are RNA molecules, and the corresponding dsDNA targets can be supplied by users, who therefore gain complete control over the sequence, length, and chemical properties of the dsDNA targets. As one important aspect of the invention, the at least one PAM is now encoded into the dsDNA target, circumventing PAM requirements in the substrates of RNA to be detected, and thus providing PAM-independent detection. The inventors designate the resulting approach for Cas-based, in particular Cas12-based, RNA detection via collateral nuclease activity "PUMA".

The inventor's previous work showed that Cas9 tracrRNAs can also hybridize to certain cellular RNAs harboring a repeat-like sequence, converting that region into a gRNA used by Cas9 (29). In line with this finding, the inventors and others reprogrammed the hybridizing region of the Cas9 tracrRNA to match to a specific RNA-of-interest that preserves the structure and necessary sequence features of the natural RNA duplex (29-31). The region of the RNA-of-interest then becomes a gRNA that directs sequence-specific DNA targeting activity by Cas9. This RNA-sensing capability of reprogrammed tracrRNAs (Rptrs) is the basis for the *in vitro* multiplexed RNA detection platform LEOPARD (29), the *in vivo* transcriptional sensor AGATHA (31) and the *in vivo* single-cell RNA recording platform TIGER (30). In all cases, a PAM was appended to the target sequence; accordingly, the Cas9 nuclease's particular PAM could be disregarded when selecting the region of an RNA to sense. These findings created the opportunity to extend tracrRNA engineering to Cas12, combining PAM-free RNA detection previously associated with Cas9 nucleases and collateral cleavage as well as other features uniquely associated with the diverse set of tracrRNA-dependent Cas12 nucleases.

The inventors demonstrated the utility of PUMA by detecting and differentiating five different bacterial pathogens using a single universal Rptr (see below). The inventor's tracrRNA reprogramming strategy provides a streamlined solution to detection challenges faced by Cas12 and will further advance CRISPR-based diagnostics at large.

While developing PUMA, the inventors surprisingly found that shortened and/or partially or fully processed dsDNA targets boost the trans-cleavage activity and increase detection speed. The inventors further found that this truncated dsDNA target strategy also applies to other Cas, particularly Cas12, -based diagnostics, with the dsDNA targets being processed by, for example, Type IIS restriction enzymes. Moreover, introducing synthetic mutations into the dsDNA targets allowed for RNA detection at the same single-nucleotide resolution as applied previously (9, 29).

Kaminski, M.M., In: CRISPR-based diagnostics. Nat Biomed Eng 5, 643-656 (2021). https://doi.org/10.1038/s41551-021-00760-7) describe that the accurate and timely diagnosis of disease is a prerequisite for efficient therapeutic intervention and epidemiological surveillance. Diagnostics based on the detection of nucleic acids are among the most sensitive and specific, yet most such assays require costly equipment and trained personnel. Recent developments in diagnostic technologies, in particular those leveraging clustered regularly interspaced short palindromic repeats (CRISPR), aim to enable accurate testing at home, at the point of care and in the field. In this Review, they provide a rundown of the rapidly expanding toolbox for CRISPR-based diagnostics, in particular the various assays, preamplification strategies and readouts, and highlight their main applications in the sensing of a wide range of molecular targets relevant to human health.

It was therefore established as a second important aspect in the context of the present invention, that a shorter nucleic acid encoding the target sequence is sufficient in order to increase the collateral nuclease activity of the Cas nuclease in the method, even if the actual target sequence (i.e., sequence complementary to the gRNA guide) is still present in full-length. Cas12 nucleases cleave their targets near the 3' end of the target nucleic acid, normally leaving sufficient target sequence to remain base paired with the gRNA guide. Given this, truncating or shortening the target nucleic acid while actually improving the collateral activity was surprising, since a truncated or shortened DNA target nucleic acid is presented, which normally should exhibit reduced DNA cleavage.

In the context of the present invention, the at least one dsDNA target nucleic acid is targeted or bound by a Cas nuclease enzyme. The targeting involves a binding of the nuclease enzyme to a binding site, either directly, or with the help of a secondary enzyme or domain, wherein cleaving comprises cleaving with a suitable enzyme. Cleaving in the context of the present invention can be achieved using Cas nucleases, meganucleases, zinc-finger nucleases, and TALE nucleases, or a restriction enzyme. If the cleavage site is a restriction site, preferably a IIS restriction enzyme, such as, for example, Acul, Bbyl, or NmeAIII, is used.

Preferred is the method according to the present invention, wherein the shortened at least one dsDNA target nucleic acid is arranged as 5'-upstream extending sequence - PAM - target sequence - downstream extending sequence-3', 5'-upstream extending sequence - PAM - target sequence-3', or 5'-upstream extending sequence - PAM - shortened target sequence-3'. These arrangements preferably apply to all DNA-targeting Cas12 nucleases. Using a shortened downstream extending sequence already increases the collateral nuclease activity of the Cas nuclease, nevertheless, the shortening or truncation can extend into the target nucleic acid sequence itself, as described herein.

Another preferred aspect of the present invention relates to the method according to the present invention, wherein the TS sequence has a length of between about 15 to 40 nt, preferably between about 18 to 30 nt, more preferred about 20 to 24 nt.

Yet, another preferred aspect of the present invention relates to the method according to the present invention, wherein the binding or targeting of the at least one dsDNA target nucleic acid by a Cas nuclease enzyme is dependent from an RNA molecule to be detected, preferably an mRNA molecule to be detected.

Another particularly preferred aspect of the present invention relates to the method according to the present invention, wherein the method is performed in the absence of designed or modified gRNA molecules. The inventors surprisingly found that the absence of a gRNA molecule (i.e., the reaction lacks a standard guide RNA when using Rptr) that is normally present for a standard Cas or Cas12-based dsDNA detection assays reduces the background collateral activity that can compromise assay sensitivity. Hence, this problem was overcome as well.

Another preferred aspect of the present invention then relates to a dsDNA target nucleic acid molecule for a CRISPR diagnostic method comprising a target strand (TS) sequence comprising a Cas nuclease cleavage site and an opposite non-target strand (NTS) sequence, a sequence extending downstream from said TS sequence, a protospacer adjacent motif (PAM) sequence directly or adjacently located upstream or downstream from said TS sequence, and a sequence extending upstream from said TS sequence, wherein the sequence extending downstream has a length of 30 base pairs or less, and is preferably missing and if the downstream extended sequence is missing, the TS sequence is truncated for between 0 to 8, preferably between 2 to 4, nucleotide bases and/or the NTS sequence is truncated for between 0 to 10, preferably between 6 to 8, nucleotide bases.

Preferred is the dsDNA target nucleic acid molecule for a CRISPR diagnostic method according to the present invention, wherein the length, such as overall length of the at least one dsDNA target nucleic acid is between about 120 and about 24 bp, preferably between about 100 and about 30 bp, more preferably at between about 60 and about 35 bp.

Preferred is the dsDNA target nucleic acid molecule for a CRISPR diagnostic method according to the present invention, wherein the dsDNA target nucleic acid molecule is cleaved by a restriction enzyme that creates a blunt or overhang end, such as an IIS restriction enzyme, such as, for example, Acul, Bbyl, or NmeAIII. Preferably, this creates an overhang sequence at one end of the target nucleic acid molecule. More preferred is the dsDNA target nucleic acid molecule for a CRISPR diagnostic method according to the present invention, wherein the dsDNA target nucleic acid is arranged as 5'-upstream extending sequence - PAM - target sequence - downstream extending sequence-3', 5'-upstream extending sequence - PAM - target sequence-3', or 5'-upstream extending sequence - PAM - shortened target sequence-3'.

Another preferred aspect of the present invention then relates to the use of the dsDNA target nucleic acid molecule according to the present invention in a CRISPR diagnostic method for detecting a specific RNA molecule, preferably an mRNA molecule. Preferred is the use according to the present invention, wherein the method is performed in vivo or in vitro, and optionally the at least one specific RNA molecule is detected in a sample.

Another preferred aspect of the present invention then relates to a kit for performing the method according to the present invention, comprising at least one dsDNA target nucleic acid molecule according to the present invention, together with suitable auxiliary agents, and optionally at least one of i) a Cas enzyme binding to dsDNA nucleic acid molecules, ii) a restriction enzyme, such as an IIS restriction enzyme, such as, for example, Acul, Bbyl, or NmeAIII, iii) at least one marker nucleic acid comprising a suitable marker, for example comprising a fluorescence marker, and iv) instructions for use. Particularly preferred is the kit according to the present invention comprising suitable materials for a colorimetric readout or a lateral flow assay. See, for example, Kaminski, M.M., et al. CRISPR-based diagnostics. Nat Biomed Eng 5, 643-656 (2021). https://doi.org/10.1038/s41551-021-00760-7.

Another preferred aspect of the present invention then relates to the use of the kit according to the present invention for detecting a specific RNA molecule, preferably an mRNA molecule in a CRISPR diagnostic method or assay as described herein.

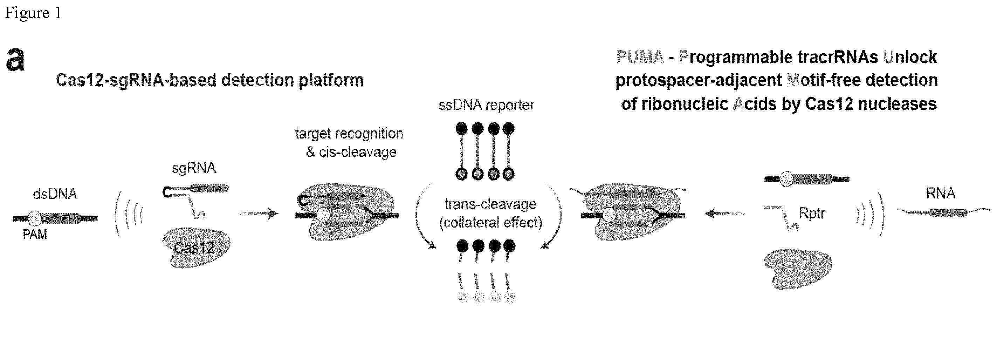

In summary, here, the inventors report a novel strategy to achieve direct, PAM-free detection of RNA by reprogramming tracrRNAs associated with a diverse set of Cas12 nucleases (Fig. 1a). The inventors call this approach PUMA, for Programmable tracrRNAs Unlock protospacer-adjacent Motif-free detection of ribonucleic Acids by Cas12 nucleases. The inventors first demonstrate the reprogrammability of tracrRNAs associated with Cas12b, Cas12e and Cas12f1 nucleases. Subsequently, the inventors found that RNA detection could be enhanced by using shorter and processed DNA targets. The inventors further show that the absence of a gRNAnormally present for standard Cas12-based dsDNA detection assays reduces background collateral activity that can compromise assay sensitivity. Finally, as an example the inventors demonstrate the utility of PUMA by detecting and differentiating five different bacterial pathogens using a single universal Rptr. The inventor's tracrRNA reprogramming strategy provides a streamlined solution to detection challenges faced by Cas12 and could further advance CRISPR-based diagnostics at large.

As mentioned above, in a first aspect of the present invention, the above object is solved by a CRISPR diagnostic method for detecting at least one specific dsDNA target nucleic acid, generally comprising the steps of binding or targeting the at least one dsDNA target nucleic acid by a Cas nuclease enzyme, and subsequently cleaving of at least one marker nucleic acid by collateral nuclease activity of the Cas nuclease enzyme.

CRISPR-based diagnostics is a molecular diagnostic system that uses the CRISPR (Clustered Regularly Interspaced Short Palindromic Repeats) technology to detect specific DNA or RNA sequences in a sample (see, for example, Nafian, F., et al. CRISPR-Based Diagnostics and Microfluidics for COVID-19 Point-of-Care Testing: A Review of Main Applications. Mol Biotechnol65, 497-508 (2023). https://doi.org/10.1007/s12033-022-00570-5).

An important focus of the present invention lays on an at least one specific dsDNA (ds: double-stranded) target nucleic acid that is cleaved by a Cas nuclease enzyme. The at least one specific dsDNA target nucleic acid comprises a target strand (TS) sequence comprising a Cas nuclease cleavage site.

Unless shortened or truncated (see below), the TS sequence has a standard length, and preferably has a length of between about 15 to 40 nt (nt: nucleotides or bases), preferably between about 18 to 30 nt, more preferred about 20 to 24 nt (see, for example, for Cas12i, Zhang, H., et al. Mechanisms for target recognition and cleavage by the Cas12i RNA-guided endonuclease. Nat Struct Mol Biol 27, 1069-1076 (2020). https://doi.org/10.1038/s41594-020-0499-0). The target strand (TS) sequence may be modified, such as, for example mutated, for example to improve the targeting and/or cleaving activity of the nuclease enzyme.

The target strand (TS) sequence and a non-target strand (NTS) sequence then form a TS:NTS duplex, creating one part of the dsDNA target nucleic acid.

The dsDNA target nucleic acid then furthermore comprises at least one protospacer adjacent motif (PAM) sequence directly located upstream and/or downstream of the TS:NTS duplex. The PAM sequence is located directly or adjacent upstream and/or or directly or adjacent downstream from the TS sequence. There are cases where there are non-critical bases (n's) at the beginning of the PAM. The dsDNA target nucleic acid may comprise more than one PAM sequence, preferably having different Cas nuclease specificities.

The dsDNA target nucleic acid then furthermore optionally comprises a pre-selected nucleic acid sequence extending upstream and/or downstream from the PAM and/or TS:NTS duplex.

Importantly, the dsDNA target nucleic acid is shortened, which - as was surprisingly found - allows for a faster detection reaction without loss of sensitivity. Therefore, before the step of binding the dsDNA target nucleic acid molecule by the Cas nuclease enzyme, the sequence extending from the TS:NTS duplex is shortened to about 30 base pairs or less in length. Preferably, the extending sequence, either upstream and/or downstream is eliminated. If then the downstream extended sequence is eliminated, the TS sequence may be further shortened or truncated between 0 to 8, preferably between 2 to 4, nucleotide bases and/or the NTS sequence is truncated between 0 to 10, preferably between 6 to 8, nucleotide bases.

Preferred is the method according to the present invention, wherein the overall length of the shortened at least one dsDNA target nucleic acid is between about 120 and about 24 bp, preferably between about 100 and about 30 bp, more preferably at between about 60 and about 35 bp.

In diagnostic methods that involve nucleic acid amplification of the target, preferably the shortening the dsDNA target nucleic acid molecule comprises the steps of introducing a cleavage site into the dsDNA target nucleic acid molecule and subsequent enzymatic cleavage, preferably using DNA amplification with primers that are modified to include the cleavage site. Preferably, a restriction site is introduced into the dsDNA target nucleic acid molecule and subsequent restriction enzyme digestion occurs. Preferably DNA amplification is performed with primers that are modified to include the restriction site, wherein the restriction site is preferably recognized by an IIS restriction enzyme, such as, for example, Acul, Bbyl, or NmeAIII.

Cleaving comprises cleaving with a deoxyribonuclease such as an enzyme selected from restriction enzymes, Cas nucleases, meganucleases, zinc-finger nucleases, and TALE nucleases, and wherein preferably the cleavage site is a restriction site that is preferably recognized by an IIS restriction enzyme, such as, for example, Acul, Bbyl, or NmeAIII. Preferably, the Cas nuclease enzyme is selected from a Cas enzyme binding to dsDNA nucleic acid molecules, such as, for example, Cas9 or Cas12, in particular Cas12a, Cas12b, Cas12c, Cas12d, Cas12e, Cas12f1, Cas12g, Cas12i, and Cas12l.

The shortened DNA target or the substrate for cleavage can be generated through a preamplification reaction starting from a pool of DNA or with reverse transcription for RNA targets. The primers set the bounds or borders of the amplified DNA and can integrate cleaving sequences, such as a restriction site.

In other reactions and assay formats, the dsDNA target nucleic acid molecule is provided as a synthesized shortened molecule. In these cases, the dsDNA target nucleic acid molecule may be provided chemically or enzymatically synthesized. The dsDNA target can also be chemically modified, such as with modifications to the phosphate backbone.

Exemplary shortened at least one dsDNA target nucleic acids in the method according to the present invention may be arranged as
5'-upstream extending sequence - PAM - target sequence - downstream extending sequence-3',
5'-upstream extending sequence - PAM - target sequence-3', or
5'-upstream extending sequence - PAM - shortened target sequence-3'.

Nevertheless, the present invention also includes molecules with different orientations, as long as these molecule function as dsDNA target nucleic acid molecule for a CRISPR diagnostic method according to the present invention.

In the method according to the present invention, upon binding and cleaving the dsDNA target nucleic acid molecule, the Cas nuclease enzyme subsequently cleaves at least one marker nucleic acid by collateral nuclease activity. Collateral cleavage activity of a single-stranded DNA substrate occurs that yields a marker, such as a fluorescence signal. The fluorescence accumulates faster with a shortened DNA target according to the invention than a longer DNA target. Preferred is a method according to the present invention, wherein cleaving the at least one marker nucleic acid is detected, for example comprising a fluorescence marker. Markers can be quenchers, dyes, enzymes, isotopes, weight markers, metal complexes, immunomarkers, and the like.

The at least one marker nucleic acid is a single stranded nucleic acid selected from ssDNA, RNA, and mixtures or derivatives thereof. Examples are DNA-RNA mixes or PNA including nucleic acids, as well as nucleic acids containing modified bases.

Yet, another preferred aspect of the present invention relates to the method according to the present invention, wherein the binding or targeting of the at least one dsDNA target nucleic acid by a Cas nuclease enzyme is dependent from an RNA molecule to be detected, preferably an mRNA molecule to be detected.

Another particularly preferred aspect of the present invention relates to the method according to the present invention, wherein the method is performed in the absence of designed or modified gRNA molecules. The inventors surprisingly found that the absence of a gRNA molecule (i.e., the reaction lacks a standard guide RNA when using Rptr) that is normally present for a standard Cas or Cas12-based dsDNA detection assays reduces the background collateral activity that can compromise assay sensitivity Hence, this problem was overcome as well.

Another particularly preferred aspect of the present invention relates to the method according to the present invention, wherein the method is performed in vivo or in vitro, and optionally the at least one specific dsDNA target nucleic acid is detected in a sample.

Another aspect of the present invention then relates to a dsDNA target nucleic acid molecule for a CRISPR diagnostic method comprising a target strand (TS) sequence comprising a Cas nuclease cleavage site and an opposite non-target strand (NTS) sequence, a protospacer adjacent motif (PAM) sequence directly located upstream or downstream the TS:NTS duplex, and a sequence extending upstream and/or downstream from the PAM and/or TS:NTS duplex, wherein the sequence extending downstream has a length of about 30 base pairs or less, and is preferably missing and if the downstream extended sequence is missing, the TS sequence is truncated for between 0 to 8, preferably between 2 to 4, nucleotide bases and/or the NTS sequence is truncated for between 0 to 10, preferably between 6 to 8, nucleotide bases.

The at least one specific dsDNA target nucleic acid comprises a target strand (TS) sequence comprising a Cas nuclease cleavage site.

Unless shortened or truncated (see below), the TS sequence has a standard length, and preferably has a length of between about 15 to 40 nt (nt: nucleotides or bases), preferably between about 18 to 30 nt, more preferred about 20 to 24 nt (see, for example, for Cas12i, Zhang, H., et al. Mechanisms for target recognition and cleavage by the Cas12i RNA-guided endonuclease. Nat Struct Mol Biol 27, 1069-1076 (2020). https://doi.org/10.1038/s41594-020-0499-0). The target strand (TS) sequence may be modified, such as, for example mutated, for example to improve the targeting and/or cleaving activity of the nuclease enzyme.

The target strand (TS) sequence and a non-target strand (NTS) sequence then form a TS:NTS duplex, creating one part of the dsDNA target nucleic acid.

The dsDNA target nucleic acid then furthermore comprises at least one protospacer adjacent motif (PAM) sequence directly located upstream and/or downstream of the TS:NTS duplex.

The PAM sequence is located directly or adjacent upstream and/or or directly or adjacent downstream from the TS sequence. Nevertheless, the PAM may be about 2-6 nucleotides downstream of the DNA sequence targeted by the Rptr. The dsDNA target nucleic acid may comprise more than one PAM sequence, preferably having different Cas nuclease specificities.

The dsDNA target nucleic acid then furthermore optionally comprises a pre-selected nucleic acid sequence extending upstream and/or downstream from the PAM and/or TS:NTS duplex.

Importantly, the dsDNA target nucleic acid is shortened, which - as was surprisingly found - allows for a faster detection reaction without loss of sensitivity. Therefore, before the step of binding the dsDNA target nucleic acid molecule by the Cas nuclease enzyme, the sequence extending from the TS:NTS duplex is shortened to about 30 base pairs or less in length. Preferably, the extending sequence, either upstream and/or downstream is eliminated. If then the downstream extended sequence is eliminated, the TS sequence may be further shortened or truncated between 0 to 8, preferably between 2 to 4, nucleotide bases and/or the NTS sequence is truncated between 0 to 10, preferably between 6 to 8, nucleotide bases.

Preferred dsDNA target nucleic acid molecule for a CRISPR diagnostic method according to the present invention, wherein the overall length of the shortened at least one dsDNA target nucleic acid is between about 120 and about 24 bp, preferably between about 100 and about 30 bp, more preferably at between about 60 and about 35 bp.

Preferably, the shortening the dsDNA target nucleic acid molecule comprises the steps of introducing a cleavage site into the dsDNA target nucleic acid molecule and subsequent enzymatic cleavage, preferably using DNA amplification with primers that are modified to include the cleavage site. Preferably, a restriction site is introduced into the dsDNA target nucleic acid molecule and subsequent restriction enzyme digestion occurs. Preferably DNA amplification is performed with primers that are modified to include the restriction site, wherein the restriction site is preferably recognized by an IIS restriction enzyme, such as, for example, Acul, Bbyl, or NmeAIII.

In other reactions and assay formats, the dsDNA target nucleic acid molecule is provided as a synthesized shortened molecule. In these cases, the dsDNA target nucleic acid molecule may be provided chemically or enzymatically synthesized.

Exemplary shortened at least one dsDNA target nucleic acids in the method according to the present invention may be arranged as
5'-upstream extending sequence - PAM - target sequence - downstream extending sequence-3',
5'-upstream extending sequence - PAM - target sequence-3', or
5'-upstream extending sequence - PAM - shortened target sequence-3'.

Nevertheless, the present invention also includes molecules with different orientations, as long as these molecule function as dsDNA target nucleic acid molecule for a CRISPR diagnostic method according to the present invention.

One exemplary shortened dsDNA target nucleic acid molecule is one that minimally contains an upstream sequence, a PAM, and a DNA target with different extents of truncation. The upstream sequence would be a minimum of 5 bps and preferably 12 bps but can be much longer. The PAM is unique to the selected Cas nuclease. The DNA target, which is normally about 24 bps, can be shortened on either or both the target strand and the non-target strand. The DNA target can also be extended with any sequence for different lengths, from one nt to over a hundred nts. Preferably, the DNA target is not extended and contains a 6-nt truncation on the non-target strand and 2-4-nt truncation on the target strand.

Another aspect of the present invention then relates to the use of the dsDNA target nucleic acid molecule according to the present invention in a CRISPR diagnostic method for detecting a specific RNA molecule, preferably an mRNA molecule, a non-coding RNA, a viral RNA molecule, or an in vitro-transcribed RNA from a DNA to be detected. Preferred is the use according to the present invention, wherein the method is performed in vivo or in vitro, and optionally the at least one specific RNA molecule is detected in a sample.

Another aspect of the present invention then relates to a kit for performing the method according to the present invention, comprising at least one dsDNA target nucleic acid molecule according to the present invention together with suitable auxiliary agents, and optionally at least one of i) a Cas enzyme binding to dsDNA nucleic acid molecules, ii) a restriction enzyme, such as an IIS restriction enzyme, such as, for example, Acul, Bbyl, or NmeAIII, iii) at least one marker nucleic acid comprising a suitable marker, for example comprising a fluorescence marker, and iv) instructions for use.

Preferred is a diagnostic kit according to the present invention, wherein the dsDNA target nucleic acid molecule is immobilized on a solid matrix. Nucleic acids of the assay can be labeled (e.g. with a fluorescent label, an enzymatic label, a mass label, or the like) for the diagnosis.

Preferred is a diagnostic kit according to the present invention, comprising suitable materials for a lateral flow assay. Lateral flow assays are known in the art (See, for example, Kaminski, M.M., et al. CRISPR-based diagnostics. Nat Biomed Eng 5, 643-656 (2021). https://doi.org/10.1038/s41551-021-00760-7). Other suitable assay formats are fluorescence and calorimetric assays, that are well-known to the person of skill.

Another aspect of the present invention then relates to the use of the kit according to the present invention for detecting a specific RNA molecule, preferably an mRNA molecule in a CRISPR diagnostic method.

In the context of the present invention, the term "about" shall indicate a deviation of +/- 10 % from the given number, unless indicated otherwise.

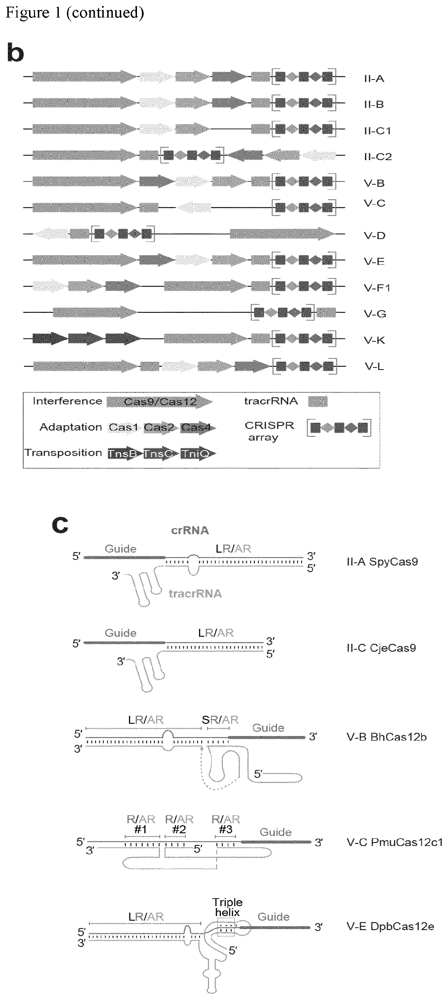

Type V CRISPR-Cas systems comprise numerous systems that involve tracrRNAs that could be amenable to tracrRNA engineering. Of the 14 subtypes of type V systems defined to-date, 8 (associated with Cas12b, Cas12c, Cas12d, Cas12e, Cas12f1, Cas12g, Cas12k and Cas12l) exclusively rely on a tracrRNA for gRNA biogenesis (Fig. 1b) (5, 6, 12, 32-41). Apart from collateral cleavage activity (9, 10, 33, 36, 42), some or all of these DNA-targeting nucleases possess features distinct from more traditional Cas9 nucleases, such as pre-crRNA processing (36, 43, 44), compact nucleases 37-40, 42, 45), higher optimal temperatures (10, 34, 46), crRNA-guided transposition (35, 47) and T-rich and C-rich PAM recognition (6, 12, 41, 43, 44).

TracrRNA reprogramming involves engineering the anti-repeat region to hybridize with an RNA-of-interest while maintaining the essential sequence and structural features of the natural repeat/anti-repeat (R/AR) duplex recognized by the Cas nuclease. While Cas9-associated RNA duplexes form a simple 25-40 bp stem typically interrupted by small bulge (48-50), Cas12-associated RNA duplexes adopt distinct and more complicated conformations (Fig. 1b-c). In addition to a long repeat/anti-repeat (LR/AR) stem often containing an intervening bulge, the reported duplexes associated with Cas12b, Cas12f1, Cas12g and Cas12l also possess a pseudoknot that includes a 5-7 bp short repeat/anti-repeat (SR/AR) stem (6, 15, 41, 51-54). For Cas12e and Cas12k, the reported RNA duplexes possess a 3-bp triple helix formed by two portions of the anti-repeat sandwiching the repeat in addition to the bulged LR/AR stem (33, 47, 55, 56) (Figs. 1c). Finally, for Cas12c, the reported RNA duplexes form three 4-7 bp disjoint R/AR stems (43) (Fig. 1c). Given the diversity and complexity of these RNA duplexes, the inventors asked how well can the RNA duplexes associated with these diverse tracrRNAs be reprogrammed for RNA detection.

The inventor's previous work revealed that Cas9 tracrRNAs can also hybridize to certain cellular RNAs harboring a repeat-like sequence, converting that region into a gRNA used by Cas9 (29). In line with this discovery, the inventors and others reprogrammed the hybridizing region of the Cas9 tracrRNA to match to a specific RNA-of-interest that preserves the structure and necessary sequence features of the natural RNA duplex (29-31). The region of the RNA-of-interest then becomes a gRNA that directs sequence-specific DNA targeting activity by Cas9. This RNA-sensing capability of reprogrammed tracrRNAs (Rptrs) was the foundation for the *in vitro* multiplexed RNA detection platform LEOPARD (29), the *in vivo* transcriptional sensor AGATHA (31) and the *in vivo* single-cell RNA recording platform TIGER (30). In all cases, a PAM was appended to the target sequence; accordingly, the Cas9 nuclease's particular PAM could be disregarded when selecting the region of an RNA to sense. These advances create the opportunity to extend tracrRNA engineering to Cas12, combining PAM-free RNA detection previously associated with Cas9 nucleases and collateral cleavage as well as other features uniquely associated with the diverse set of tracrRNA-dependent Cas12 nucleases.

Here, the inventors report a novel strategy to achieve direct, PAM-free detection of RNA by reprogramming tracrRNAs associated with a diverse set of Cas12 nucleases (Fig. 1a). The inventors call this approach PUMA, for Programmable tracrRNAs Unlock protospacer-adjacent Motif-free detection of ribonucleic Acids by Cas12 nucleases. The inventors first demonstrate the reprogrammability of tracrRNAs associated with Cas12b, Cas12e and Cas12f1 nucleases. Subsequently, the inventors found that RNA detection could be enhanced by using shorter and processed DNA targets.

The inventors further show that the absence of a gRNA normally present for standard Cas12-based dsDNA detection assays reduces background collateral activity that can compromise assay sensitivity. Finally, the inventors demonstrate the utility of PUMA by detecting and differentiating five different bacterial pathogens using a single universal Rptr. The inventor's tracrRNA reprogramming strategy provides a streamlined solution to detection challenges faced by Cas12 and could further advance CRISPR-based diagnostics at large.

Therefore, the present invention in one aspect relates to the reprogramming or modification of tracrRNAs associated with diverse type V CRISPR-Cas systems to address two general challenges with traditional Cas12-based detection technologies: direct RNA detection and PAM requirements. Rptrs allow dsDNA-targeting Cas12 nucleases to directly detect RNA by binding and converting RNAs to be detected into guide RNAs for dsDNA targeting. The detected substrates are RNA, and the corresponding dsDNA targets can be supplied by users, who thus gain complete control over the sequence, length, and chemical properties of the dsDNA targets. As one aspect of the invention, the PAM is encoded into the dsDNA target, circumventing PAM requirements in the substrates of RNA to be detected. The inventors designate the resulting approach for Cas12-based RNA detection via collateral activity "PUMA".

While developing PUMA, the inventors furthermore and importantly found that shortened and processed dsDNA targets boost the trans-cleavage activity and increase detection speed. The inventors found that this truncated dsDNA target strategy also applies to other Cas12-based diagnostics, with the target strand in the dsDNA targets being processed by Type IIS restriction enzymes. Moreover, introducing synthetic mutations into the dsDNA targets allowed for RNA detection at a single-nucleotide level as applied previously (9, 29).

One unique property of PUMA is the consistently low background collateral activity compared to traditional Cas12-based detection technologies. For these traditional technologies, the background activities varied substantially among different sgRNAs, indicating that the guide sequence could be a key factor determining the leaky activity. The sgRNA induces conformational changes in the nuclease, exposing the RuvC active site and activating trans-cleavage activity even in the absence of a DNA target. Further structural and functional studies will reveal the underlying mechanism of this dsDNA target-independent activity, providing insights for guide RNA design to reduce or even eliminate this background activity. The inventors also recognize that promiscuous hybridization of the Rptrs to other RNAs leads to ribonucleoprotein complex formation and potential background activity, even if this possibility requires further exploration.

The LEOPARD platform achieves multiplex RNA detection in a single reaction by monitoring the sequence-specific cleavage of parallel DNA targets by Cas9 in a gel-based readout (29). Integrating the unique trans-cleavage activity of Cas12 Rptrs will enable sensitive RNA detection that exceeds that associated with LEOPARD. To do so, any combination would have to account for Cas12's non-specific trans-cleavage activity being inherently incompatible with multiplexed detection. One solution is the immobilization of DNA targets and reporters that would localize the collateral activities to certain locations, such as through microfluidics. Therefore, the development of Cas12 Rptrs creates the opportunity to combine LEOPARD and PUMA for highly multiplexed and sensitive RNA detection.

Rptrs can also be extended to other tracrRNA-encoding Type V systems to achieve alternative functions beyond RNA detection. Cas12c possesses the unique capability of tracrRNA-assisted pre-crRNA processing within the guide region. This intriguing feature can be repurposed for precise, collateral-free RNA targeting, as no trans-cleavage activity is observed for Cas12c2 (36, 43, 44). Precise RNA targeting can be achieved by using Rptrs to trigger the cis-cleavage in the resulting guide region in the target RNA. Furthermore, Rptrs can also be expanded to the Cas12k-guided transposition system, enabling transcription-dependent tissue-specific DNA integration (35, 56, 77). By using a tissue-specific mRNA bound with Rptr as the source for guide RNA, the risk of off-targeting in undesired tissues or cell types could be minimized. These examples along with PAM-free RNA detection with PUMA underscore the many technologies that will emerge from reprogramming tracrRNAs associated with Cas12 nucleases.

To date, a DNA target was never modified to impact collateral cleavage activities. At most, PAM sequences were appended to one end of the DNA or the DNA target was converted into a single-stranded version to expand the targeting scope of Cas12 nucleases. Compared to the state-of-the-art, the present invention greatly accelerates the time to generate a signal and thus a diagnostic answer and the overall sensitivity of the assay, without modifying other aspects of the CRISPR-based diagnostic process.

As an example of the invention, using a CRISPR-based diagnostic based on Cas12 (or any nuclease that recognizes dsDNA targets and subsequently elicits collateral activity), a DNA target was selected representing a desired biomarker, here a 16S rRNA sequence unique to Klebsiella pneumoniae.

In practice, for common ("traditional") CRISPR-based diagnostic methods, the corresponding DNA is amplified to form a typically 100 to 2,000 bp fragment to be detected by Cas12. In this case, the primers are modified to include a restriction site for an IIS restriction enzyme (e.g., Acul, Bbyl, NmeAIII) that cuts into the DNA target, creating a truncation. The restriction enzyme is then included following amplification to generate an overhang in line with a shortened DNA target. Different restriction sites can be incorporated using known methods, e.g., as part of PCR or isothermal amplification, in order to create the shortened/truncated DNA target. The DNA target is then recognized by a Cas12 nuclease as provided that is bound to the corresponding guide RNA, which leads to collateral cleavage of a single-stranded DNA molecular beacon, such as fluorescence, when cleaved. The fluorescence accumulation is faster for the shortened DNA than the longer/standard DNA (e.g., 100 bp to 300 bp).

In the other case of combining Cas12 with reprogrammed tracrRNAs, the DNA target is supplied as part of the reaction, while the diagnostic assay is sensing the presence of an RNA-of-interest. In this case, the DNA target is provided as a truncated version. Collateral cleavage activity of a single-stranded DNA substrate again occurs that yields a marker, such as a fluorescence signal. As above, the fluorescence accumulates faster with a shortened DNA target according to the invention than a longer DNA target.

The invention will now be described further in the following examples with reference to the accompanying figures, nevertheless, without being limited thereto.

In the accompanying figures and the accompanying sequence listing,
Figure 1 shows the framework for PAM-independent RNA detection by tracrRNA-dependent Cas12 nucleases with PUMA. a, Overview of Cas12 Rptr-based RNA detection platform. Left: Traditional Cas12-based diagnostics use sgRNAs to recognize and cut dsDNA targets, triggering trans-cleavage activity for signal visualization and amplification. However, they have PAM limitations and cannot directly detect RNA. Right, Reprogrammed tracrRNAs (Rptrs), binding and converting RNAs to be detected into guide RNAs, enable direct and PAM-free RNA detection. b, TracrRNA-encoding CRISPR-Cas systems. TracrRNA is encoded in all Type II subtypes and in 8 of 14 identified Type V subtypes, including V-B, -C, -D, -E, -F, -G, -K, and -L. The CRISPR locus architecture is based on prior work (5, 6, 12). c, Representative examples of four different duplexes formed between the tracrRNA and crRNA among tracrRNA-encoding CRISPR-Cas systems. In type II CRISPR-Cas systems, a single imperfect RNA duplex is formed between the crRNA repeat and tracrRNA anti-repeat (R/AR). In contrast, the V-B, -F, and -G systems form two duplexes (long repeat/anti-repeat duplex, LA/AR; short repeat/anti-repeat duplex, SA/AR), the V-E and -K systems form one LA/AR duplex and one triple helix, and the V-C system forms three A/AR duplexes. PmuCas12c1, Cas12c from *Parasutterella muris,* PDB: 7VYX (78).

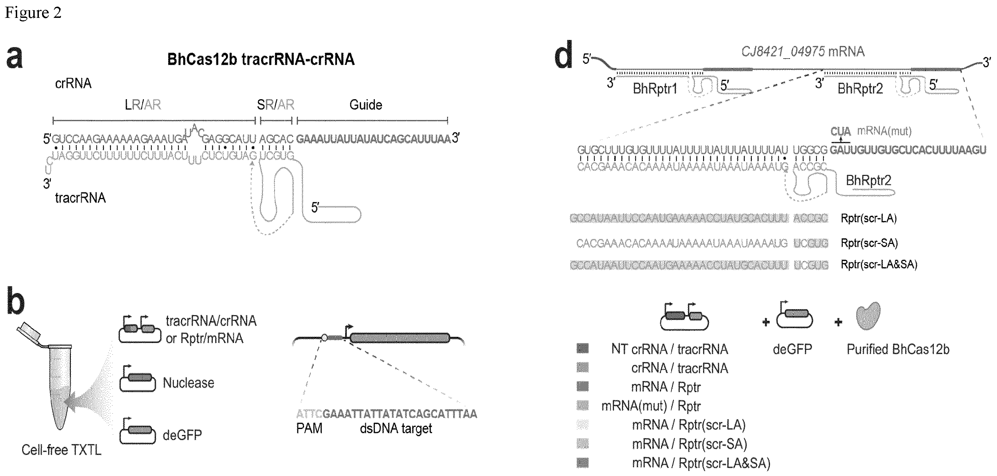

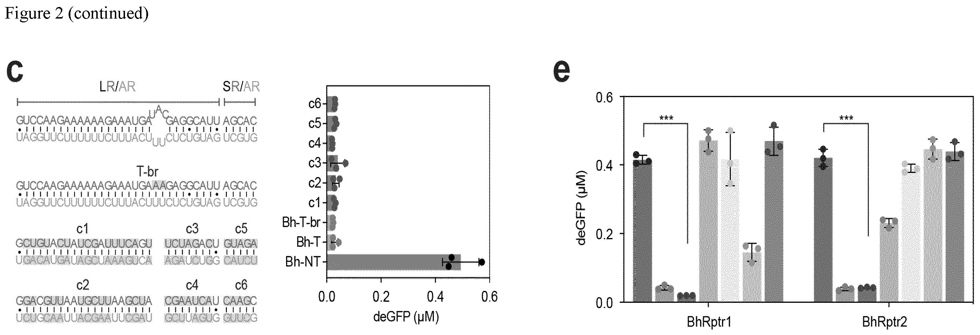

Figure 2 shows that tracrRNA reprogramming enables sequence-specific RNA detection by Cas12b. a, Predicted tracrRNA-crRNA structure for BhCas12b based on its ortholog BthCas12b (PDB: 5WTI (79)). R: AR, repeat/anti-repeat. b, Assessing Rptr functionality using the cell-free transcription-translation (TXTL) assay. Expressed Cas-guide RNA complex recognizes and cuts its dsDNA target, causing the degradation of target-encoding GFP reporter plasmid and resulting in a lower fluorescence release compared to a non-targeting guide control. c, Changeability of long and short RNA duplexes. NT, non-targeting; T-br, targeting crRNA with bulge removed. d, Reprogramming tracrRNAs to sense a *Camplybactor jejuni* transcript *CJ8421_04975* mRNA. The guide and target components are added in the form of DNA constructs, while the purified BhCas12b protein is used. mRNA(mut), mRNA with point mutations in the predicted "seed" region of the guide. Rptr(scr-LA), Rptr with the long anti-repeat sequence scrambled; Rptr(scr-SA), Rptr with the short anti-repeat sequence scrambled; Rptr(scr-LA&SA), Rptr with both long and short anti-repeat sequence scrambled. e, Rptr-guided sequence-specific dsDNA targeting in TXTL. Nucleotide changes in R/AR stems in c and d are indicated by gray boxes. Values in c and e represent the mean and standard deviation from three independently mixed TXTL reactions. *** p < 0.001.

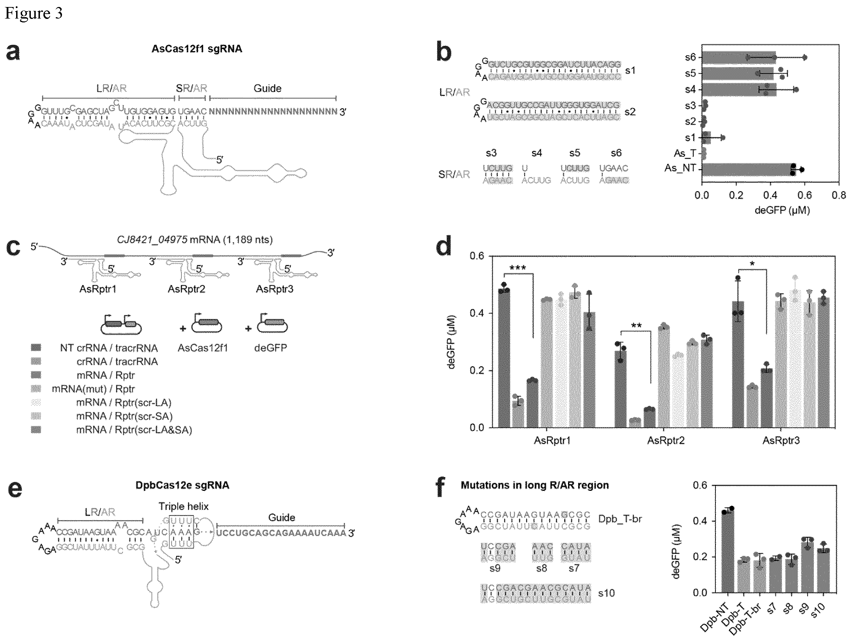

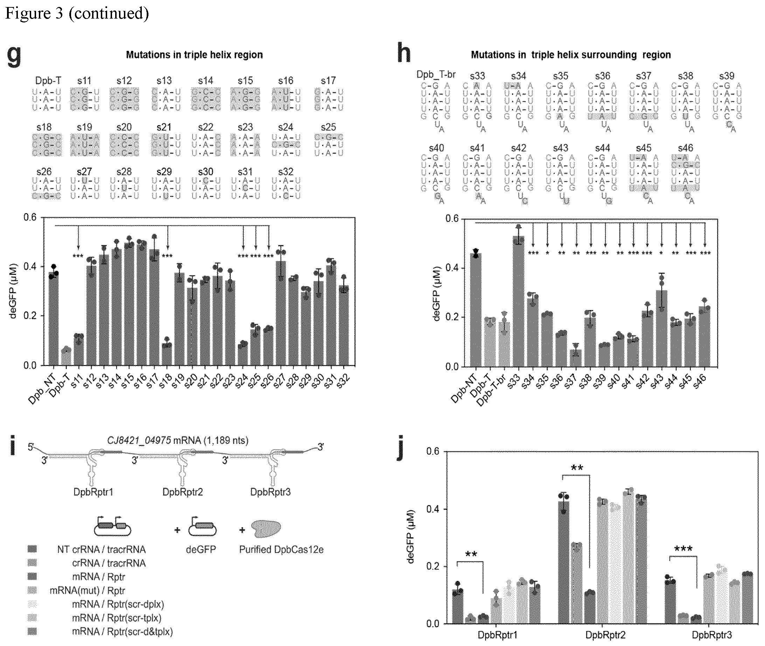

Figure 3 shows that tracrRNA reprogramming enables sequence-specific RNA detection by Cas12f1 and Cas12e. a, Predicted AsCas12f1 sgRNA structure based on Un1Cas12f1 sgRNA structure (PDB: 7C7L⁵⁴ and 7L49⁵³). b, Both the long and short RNA duplexes in AsCas12f1 sgRNA can be reprogrammed. NT, non-targeting crRNA; T, targeting crRNA. c, Sensing the *Campylobacter jejuni* transcript *CJ8421_04975* mRNA using AsCas12f1 Rptrs in TXTL. d, Results for Rptr-guided sequence-specific dsDNA targeting by AsCas12f1 in TXTL. e, Structure of DpbCas12e sgRNA (PDB: 6NY3) (33). In the triple-helix region, a cis Hoogsteen/Watson-Crick base pair is formed between the U.A and a cis Watson-Crick/Watson-Crick base pair between the A-U. f, Testing the changeability of the long R:AR region in TXTL. Dpb_T-br, targeting sgRNA with bulge and G.U wobble base pair removed. g, Testing the changeability of RNA triple-helix region in TXTL. h, Testing the changeability of RNA triple-helix surrounding region in TXTL. I, Sensing the *Campylobacter jejuni* transcript *CJ8421_04975* mRNA using DpbCas12e Rptrs in TXTL. j, Results for Rptr-guided sequence-specific dsDNA targeting by DpbCas12e in TXTL. Rptr(scr-dplx), Rptr with a scrambled anti-repeat sequence; Rptr(scr-tplx), Rptr with the RNA triple-helix sequence scrambled; Rptr(scr-d&tplx), Rptr with the RNA duplex and triple-helix sequence scrambled. Nucleotide changes in AsCas12f1 sgRNA and DpbCas12e sgRNA in b, f, g and h are indicated by gray boxes. Values in b, d, f, g, h, and j represent the mean and standard deviation from three independently mixed TXTL reactions. *: p < 0.05. **: p < 0.01. ***: p < 0.001.

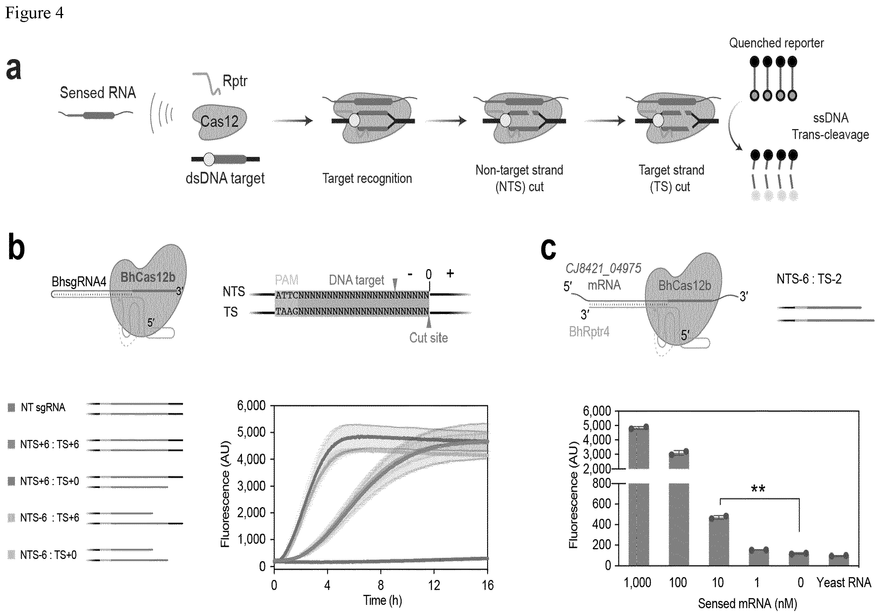

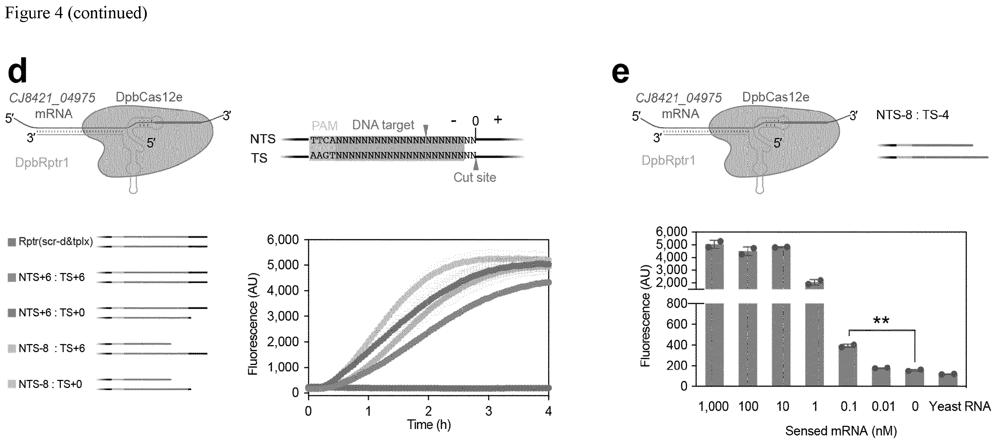

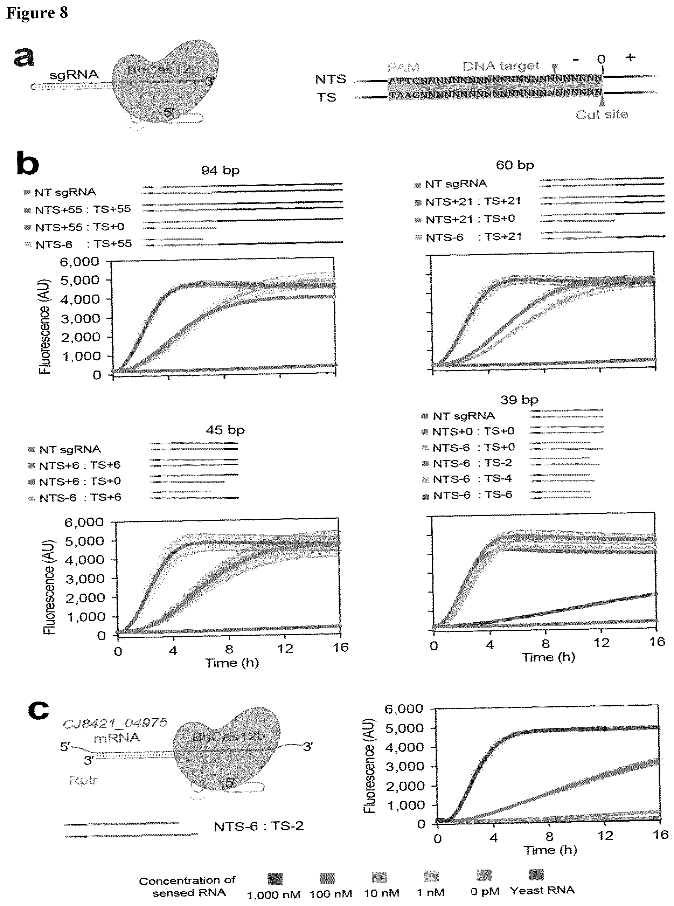

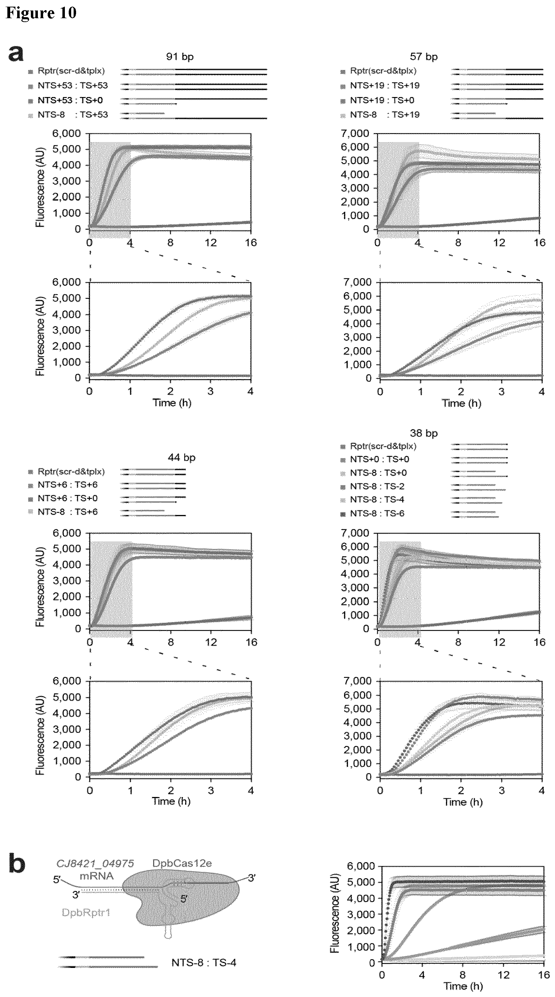

Figure 4 shows that truncating dsDNA targets boosts the rate of collateral activity. a, Schematic of *in vitro* trans-cleavage assay. The assay includes purified Cas12 nucleases, in vitro transcribed Rptrs, and linear dsDNA targets. The Cas12-guide RNA ribonucleoprotein (RNP) recognizes and cleaves its dsDNA target, which triggers non-specific cleavage activity on ssDNA. Specifically, cleavage of the non-target strand (NTS) occurs before cleavage of the target strand (TS). F, fluorophore; Q, quencher. Yellow circle, PAM; b, Impact of unprocessed or processed targets on trans-cleavage activity by BhCas12b. TS cleavage is the rate-limiting step. Red arrow, cleavage site. The cleavage site of TS is set as position 0. -, truncating the target sequence on NTS or TS. +, adding an overhang on NTS or TS. The PAM sequence is in brown and the target sequence is in blue. c, Direct detection of the full-length *CJ8421_04975* mRNA by BhCas12b, yeast RNA is added in the same mass amount as the 1000 nM mRNA to be detected, the best-performed dsDNA target NTS-6: TS-2 is used. d, Impact of unprocessed or processed targets on trans-cleavage activity by DpbCas12e. e, Direct detection of the full-length *CJ8421_04975* mRNA by DpbCas12e. Yeast RNA is added in the same mass amount as the 1000 nM mRNA to be detected, and the best-performed dsDNA target NTS-8: TS-4 is used. 16h end-point values were used to make plots for c and e. See Figures 8b and 10a for the whole time-course curves. Values in b-e represent the mean and standard deviation from two independent collateral assays. **: p < 0.01.

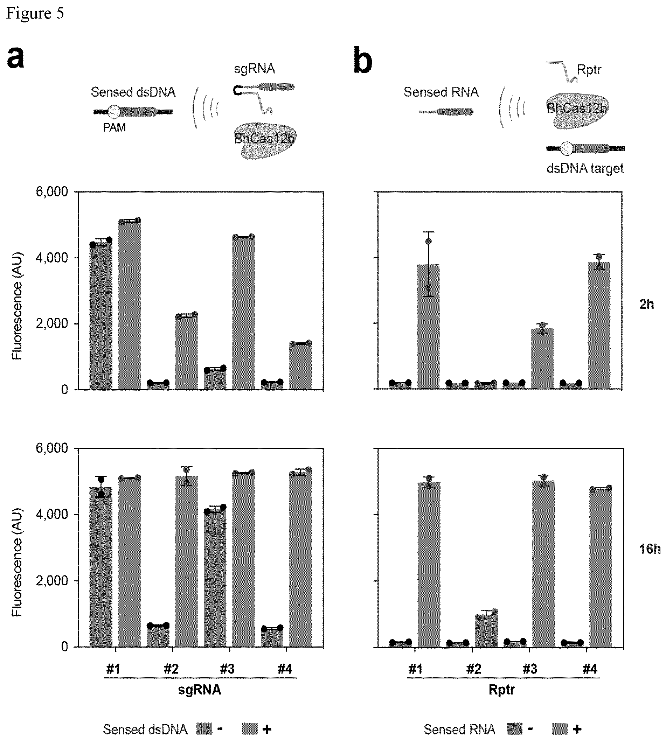

Figure 5 shows that the Rptr-based detection system exhibits reduced background collateral activity due to the absence of a traditional guide RNA. a, Some sgRNAs show substantial leaky activity even without supplying their dsDNA targets. b, No background activity is observed when the RNAs to be detected are absent. In a and b, sgRNA#1 and sgRNA#4 share the same guide sequences as those generated by Rptr#1 and Rptr#4, respectively. Values in a and b represent the mean and standard deviation from two independent collateral assays. The values at 2 hours and 16 hours are used to make the plots.

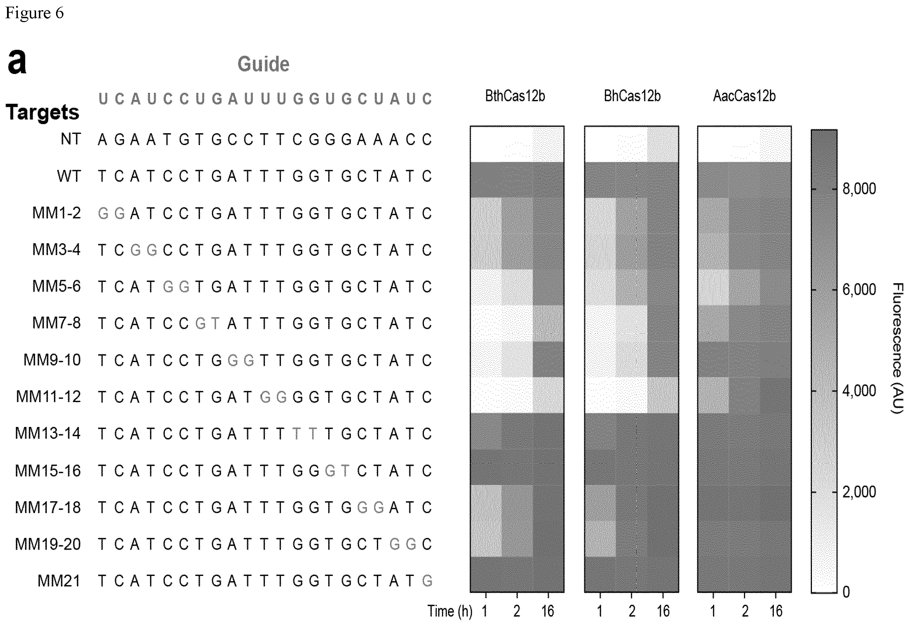

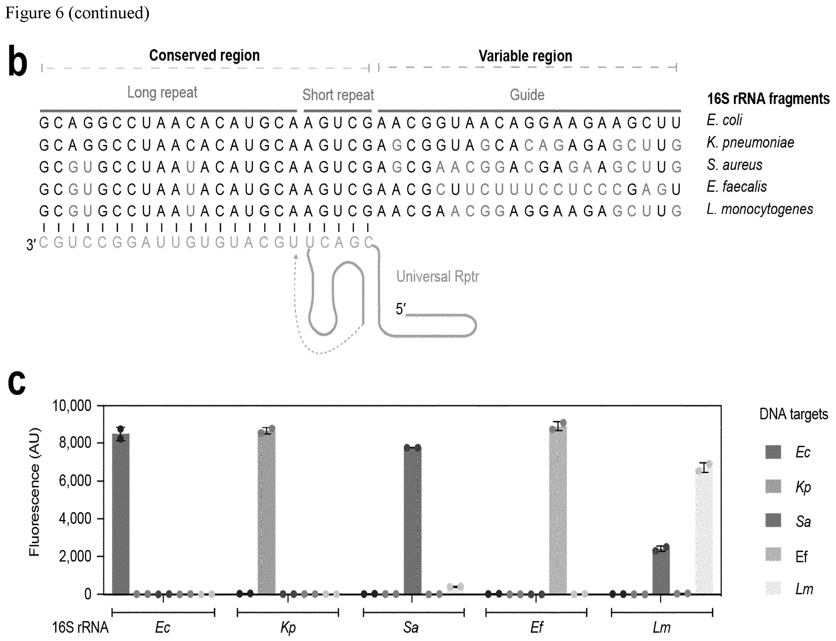

Figure 6 shows that a universal Rptr enables sequence-specific detection of 16S rRNA from different bacterial pathogens. a, Tolerance test of guide-target mismatches for three different Cas12b orthologs. Data represent the mean values from two independent collateral assays. b, 16S rRNA from five different pathogens can be differentiated using only one universal Rptr binding to the conservative region of 16S rRNA. A truncated long anti-repeat of 18 nts instead of the usual 31 nts is used in the universal Rptr. In the alignment, sequences that match the *E*. *coli* 16S rRNA are in black, while those that do not match are shown in red. c, Specific detection of pathogen 16S rRNAs with a universal Rptr and corresponding dsDNA targets. Values in c represent the mean and standard deviation from two independent collateral assays.

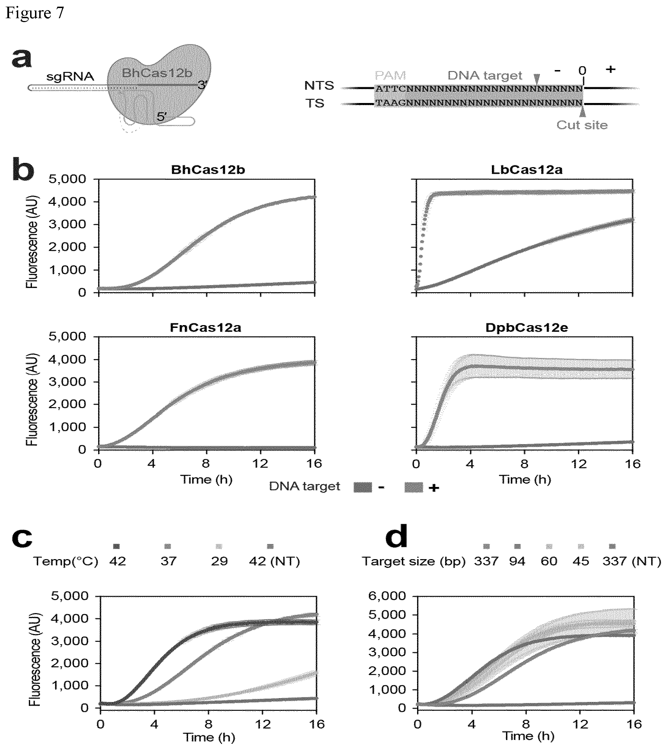

Figure 7 shows a comparison of BhCas12b's activity with other Cas12 orthologs and the optimization of its activity by altering the reaction temperature and length of dsDNA targets. a, Settings for the trans-cleavage assay. Purified BhCas12b, *in vitro* transcribed sgRNAs, linear dsDNA targets and fluorescent-labeled ssDNA reporters are used in this assay. See Fig. 4a for detailed information. b, Comparison of trans-cleavage activity among FnCas12a, BhCas12b, DpbCas12e, and LbCas12a using the same guide sequence in sgRNAs for each nuclease. c, Higher temperatures enhance the trans-cleavage activity by BhCas12b. d, Shorter dsDNA targets enhance the trans-cleavage activity by BhCas12b. NT, non-targeting sgRNA. For the observed rate constant *K_{obs}* in b, c and d. Values in b, c and d represent the mean and standard deviation from two independent collateral assays.
Figure 8 shows that truncating the dsDNA target enhances collateral cleavage by BhCas12b. a, Settings for the trans-cleavage assay. b, TS cut is the rate-limiting step, dsDNA targets with truncated TS can boost trans-cleavage activity by BhCas12b Trimming 6nt TS towards PAM (NTS-6: TS-6) dramatically reduced the activity. c, Detection of full-length *CJ8421_04975* mRNA using BhCas12b Rptr1. The data are plotted as 16h time-course curves, the plot in Fig. 4c is made using the end-point values at 16h. Yeast RNA is added in the same mass amount as the 1,000 nM mRNA to be detected. For the observed rate constant *k_{obs}* in b and c. Values in b-c represent the mean and standard deviation from two independent collateral assays.

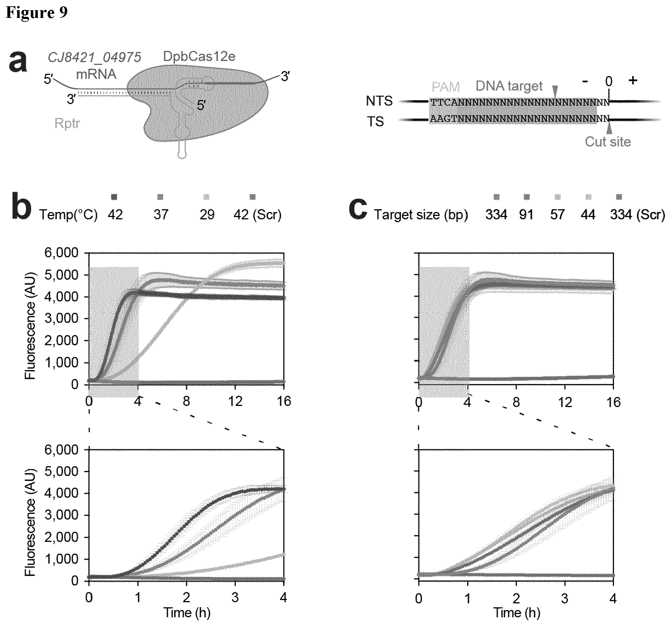

Figure 9 shows the optimizing of the trans-cleavage assay for DpbCas12e via altering reaction temperature and length of dsDNA targets. a, Settings for the trans-cleavage assay. Purified DpbCas12e, *in vitro* transcribed Rptr and *CJ8421_04975* mRNA, linear dsDNA targets and fluorescent-labeled ssDNA reporters are used in this assay. b, Higher temperatures enhance the trans-cleavage activity by DpbCas12e. c, Shorter dsDNA targets enhance the trans-cleavage activity by DpbCas12e. For the observed rate constant *k_{obs}* in b and c. Values in b-c represent the mean and standard deviation from two independent collateral assays.
Figure 10 shows that truncating the dsDNA target enhances collateral cleavage by BhCas12b. a, TS cut is the rate-limiting step, dsDNA targets with truncated TS can boost trans-cleavage activity by DpbCas12e. Different from BhCas12b, trimming 6nt TS towards PAM (NTS-8: TS-6) can be well tolerated by DpbCas 12e. b, Detection of full-length *CJ8421_04975* mRNAusing DpbCas12e Rptr1. The data are plotted as 16h time-course curves, the plot in Fig. 4e is made using the end-point values at 16h. Yeast RNA is added in the same mass amount as the 1000 nM mRNA to be detected. For the observed rate constant *k_{obs}* in a and b. Values in a-b represent the mean and standard deviation from two independent collateral assays.

### Examples

### Materials and Methods:

### Strains, oligonucleotides, and plasmids

*E. coli* strain TOP10 was used for plasmid cloning. All primers, gBlocks used in this invention were ordered from Integrated DNA Technologies. NEBuilder HiFi DNA Assembly Master Mix (New England Biolabs, cat. #E2621) was used for plasmid construction by Gibson assembly. Q5 site-directed mutagenesis kit (New England Biolabs, cat., #E0554S) was used for small insertion and nucleotide substitution. Unless otherwise specified, all nucleases used were expressed in plasmids with Cm selective marker and p15A origin-of-replication. All sgRNA, tracrRNA-crRNA, or Rptr-mRNA plasmids were expressed in plasmids with Amp selective marker and pUC origin-of-replication, and all targeted plasmids were expressed in plasmids with Kan selective marker and pSC101 origin-of-replication. *Bacillus hisashii* Cas12b (BhCas12b), *Bacillus thermoamylovorans* Cas12b (BthCas12b), *Alicyclobacillus acidoterrestris* Cas12b (AacCas12b), *Acidibacillus sulfuroxidans* Cas12f1 (AsCas12f1) and *Deltaproteobacteria* Cas12e (DpbCas12e) were PCR-amplified from plasmids pYPQ291 (Addgene ID #129671 (80)), pYPQ290 (Addgene ID #129670 (80)), pZ149-pcDNA3-BhCas12b_v4 (Addgene ID #122446 (34)), pET28a-SUMO-AsCas12f (Addgene ID #171612 (37)) and pCasX1 (Addgene ID #87685 (81)), respectively, and subcloned to a PCR-linearized version of the vector pCB583 for TXTL and plasmid clearance assay, and subcloned to a PCR-linearized version of the vector pETM11(pBR322 ori) for protein expression and purification. To construct the AsCas12f1-cytosine base editor plasmid, the dead version of AsCas12f1 was made by introducing D225A and D401A, then subjected to Gibson assembly with a PCR-linearized version of the vector pCBS1489 encoding rAPO43_1 cytidine deaminase and Uracil DNA Glycosylase Inhibitor (UGI). The resulting plasmid pCBS2127 was transformed and maintained in a low mutation strain MDS42 Meta LowMut (C-6786-10K, Scarab Genomics, LLC.). The consensus PAM (5'-ATTN-3' (34)) was used for BhCas12b, BthCas12b and AacCas12b. The consensus PAMs (5'-NTTR-3' (37) and 5'-TTCN-3' (83, 81), R = A, G) were used AsCas12f1 and DpbCas12e, respectively. To construct targeted plasmids for the TXTL assay and plasmid clearance assay, target sequences were inserted directly upstream of the constitutive OR2-OR1 promoter with the template of pCB705 using Q5 site-directed mutagenesis. Antibiotics were added where appropriate at final concentrations of 100 µg/ml for ampicillin, 34 µg/ml for chloramphenicol, and 50 µg/ml for kanamycin for *E. coli.*

### Protein expression and purification

BhCas12b, BthCas12b, AacCas12b and DpbCas12e were cloned into a pETM11 expression plasmid (T7-6×His-SUMO-nuclease), respectively. Protein purification for these nucleases was conducted as previously described with modifications (33, 34). The resulting plasmids were expressed in the BL21 Rosetta *E.coli* strain. Cultures were grown to an OD of 0.6, then expression was induced overnight at 18°C by adding IPTG at a final concentration of 0.5 mM. Cells were harvested and frozen in liquid nitrogen. The cell pellets were resuspended in a lysis buffer of 500 mM NaCl, 10% glycerol, 1 mM TCEP, 20 mM imidazole and 20 mM Tris-HCl, pH 8.0 supplemented with protease inhibitors. Cells were lysed by sonication and pelleted at 4°C and 35,000 ×g for 30 min. SUMO tag was removed by overnight digest at 4 °C with SenP2 SUMO-protease at a 1:50 weight ratio of protease to nuclease in a dialysis/ion exchange chromatography buffer of 300 mM NaCl, 10% glycerol, 1 mM DTT and 20 mM HEPES, pH 7.5. The HiTrap Heparin HP column was used to elute the cleaved proteins by gradually increasing NaCl from 0.3 M to 1 M. DpbCas12e eluted in two peaks from the HiTrap Heparin HP column, only the peak containing active protein was pooled as described previously (33). Fractions containing BhCas12b, BthCas12b, AacCas12b and DpbCas12e were pooled, concentrated and loaded onto a Superdex 200 Increase column (GE Healthcare Life Sciences) with a final storage buffer of 300 mM NaCl, 10% glycerol, 2 mM DTT, 2 mM MgCl₂, and 20 mM HEPES, pH 8. Purified proteins were concentrated to 62.2 µM (BhCas12b), 80.5 µM (BthCas12b), 19.1 µM (AacCas12b), and 35 µM (DpbCas12e) stocks and flash-frozen in liquid nitrogen before storage at -80 °C. All these protein stocks were diluted into 1µM with 1×Diluent B (300 mM NaCl, 10 mM Tris-HCl, 1 mM DTT, 0.1 mM EDTA, 500 µg/ml BSA, 50% Glycerol, pH 7.4, 25°C). The diluted proteins were stored at -20 °C and used for enzymatic cleavage assay.

### Design of Rptrs and dsDNA targets

*Campylobacter jejuni CJ8421_04975* mRNA was used as the primary template for RNAs to be detected when evaluating Rptrs. Regions conforming to the design scheme for RNAs to be detected were chosen as targets for Rptrs. Repeat regions (R) in the RNA to be detected bound by the anti-repeat region of Rptr (AR). The total length of the Rptr target was 31 nts for BhCas12b/BthCas12b/AacCas12b, 28 nts for AsCas12f1, and 37 nts for DpbCas12e. For BhCas12b, BthCas12b, AacCas12b and AsCas12f1, the long and short anti-repeat regions of the native tracrRNA were replaced with the sequences complementary to the chosen RNA targets. For DpbCas12e, the long anti-repeat region of the native tracrRNA was replaced with the sequences complementary to the chosen RNA target. Three RNA triple combinations U.A-U, C.G-C and U.G-C could be used in the RNA triple-helix region. The 20-23 nt sequences upstream of these Rptr target regions were treated as guides when designing dsDNA targets. The specific target length parallels that used for guides within sgRNAs for each Cas12 (33, 34, 37). For 16S rRNA detection of multiple pathogens, one universal Rptr was designed to base pair with the conserved region of 16S rRNA fragments from 5 different pathogens (*E.coli, Klebsiella pneumoniae, Staphylococcus aureus, Enterococcus faecalis* and *Listeria monocytogenes*).

### Cell-free transcription-translation (TXTL) reactions

TXTL reactions were conducted as previously described with minor modifications (57). The GFP variant deGFP was used as the fluorescent reporter for all TXTL assays. This variant was slightly modified for enhanced expression in TXTL (82). All DNA fragments or plasmids used in the assay were free of nucleases (DNases, RNases) and inhibitors of the TXTL machinery (e.g. EDTA, ethidium bromide, SDS, Cl- ions). BthCas12b, sgRNA variants and deGFP-encoding reporter plasmids were added into myTXTL Sigma 70 Master Mix (Arbor Biosciences, cat. #507025) at a final concentration of 1 nM, 1 nM and 0.5 nM, respectively. AsCas12f1, sgRNA/tracrRNA-crRNA/Rptr-mRNA, and deGFP-encoding reporter plasmids were added into myTXTL Sigma 70 Master Mix at a final concentration of 3 nM, 0.5 nM and 0.1 nM, respectively. Purified protein BhCas12b/DpbCas12e, sgRNA/tracrRNA-crRNA/Rptr-mRNA, and deGFP-encoding reporter plasmids were added into myTXTL Sigma 70 Master Mix at a final concentration of 100 nM, 0.5 nM and 0.1 nM, respectively. The DNA cleavage assay was performed in 5-µl reactions by measuring fluorescence on a Synergy Neo2 plate reader (BioTek) using a 96-well V-bottom plate (Corning Costar, cat. #3357) with an excitation filter of 485 nm and an emission filter of 528 nm. Time-courses were run for 16 h at 29°C for AsCas12f1, 37°C for BhCas12b, BthCas12b and DpbCas12e with an interval of 3 min between reads. The end-point values were used to make plots. A standard calibration curve produced with recombinant eGFP (Cell Biolabs, cat. #STA-201) was used to convert relative fluorescence units to the molar concentration of deGFP in the TXTL reaction.

### In vitro collateral cleavage assay

RNAs to be detected and Rptrs were encoded in double-stranded DNA fragments beginning with a T7 promoter and were transcribed using the HiScribe T7 *in vitro* transcription kit (New England Biolabs, cat. #E2040S). The transcribed RNAs were purified using RNA Clean & Concentrator-25 (Zymo Research) with residual DNA removed by In-Column DNase I treatment. The dsDNA targets were made by PCR amplification with target plasmids as templates, while the truncated and processed dsDNA targets were made through oligo annealing in 1× NEBuffer^{™} r2.1 buffer (50 mM NaCl, 10 mM Tris-HCl, 10 mM MgCl₂, 100 µg/ml Recombinant Albumin, pH 7.9. New England Biolabs, cat. #B6002). Forward and reverse oligos were annealed at a 1:1 molar ratio by heating at 95°C for 2 minutes and gradually cooling to 4°C over 30 min using a thermocycler. Purified protein BhCas12b/DpbCas12e, *in vitro* transcribed sgRNA or tracrRNA-crRNA or Rptr-mRNA, target DNA fragment, and single-strand DNA reporter (CJpr1234, 5'-FAM-TTATT-3'-IABkFQ) were added into 1× NEBuffer^{™} r2.1 buffer at a final concentration of 100 nM, 100 nM, 10 nM and 1000 nM, respectively. The trans-cleavage assay was performed in 5-µl reactions by measuring fluorescence on a Synergy Neo2 plate reader (BioTek) using a 96-well V-bottom plate (Corning Costar, cat. #3357) with an excitation filter of 485 nm and an emission filter of 528 nm. Time-courses were run for 16 h with an interval of 3 min between reads. Observed rates (*k*_{obs}) were obtained by fitting the kinetic traces to a one-phase association exponential function using Prism 9 (GraphPad Software, Inc.).

To compare background activities between sgRNA and Rptr, purified protein BhCas12b, *in vitro* transcribed sgRNA or Rptr/RNA to be detected, target DNA, and single-strand DNA reporter (CJpr1234) were added into 1× NEBuffer^{™} r2.1 buffer at a final concentration of 100 nM, 100 nM, 10 nM and 1000 nM, respectively. The absence of the dsDNA target served as the negative control for the sgRNA setting. The absence of the sense RNAs served as the negative control for the Rptr setting. Values at 2h and 16h were used to make plots.

For the mismatch tolerance test, purified protein BhCas12b/BthCas12b/AacCas12b, *in vitro* transcribed Rptr, sense RNAs, dsDNA target variants, and single-strand DNA reporter (MGhD001, 5'-FAM-TTTTT-3'-IABkFQ) were added into 1× NEBuffer^{™} r2.1 buffer at a final concentration of 100 nM, 50 nM, 50nM, 10 nM and 1000 nM, respectively. Values at 2h and 16h were used to make plots.

For 16S rRNA detection of multiple pathogens, purified protein BthCas12b, one universal Rptr, 16S rRNA fragment of different pathogens, target DNA fragments, and a single-strand DNA reporter (MGhD001) were added into 1× NEBuffer^{™} r2.1 buffer at a final concentration of 150 nM, 300 nM, 100nM, 10 nM and 1000 nM, respectively.

### Statistical analyses

All statistical comparisons were performed using a Student's T test assuming unequal variance. A one-tailed test was used in all instances based on the null hypothesis that the sample exhibited equal or worse performance than the control. Values were assumed to be normally distributed. The threshold of significance for the calculated p-values was set as 0.05.

### Results

### Reprogrammed tracrRNAs link RNA sensing and dsDNA targeting by Cas12b

The inventors started with the *Bacillus hisashii* Cas12b (BhCas12b) due to the relative simplicity of its RNA duplex comprising a 30-bp LR/AR stem with an intervening bulge, and a 5-bp SR/AR duplex between the LR/AR and the guide (34, 51) (Fig. 2a). The inventors investigated the reprogrammability of both stems using a Cas12 cleavage assay conducted with a cell-free transcription-translation (TXTL) system (57). As part of the assay, purified BhCas12b protein, a gRNA-expressing plasmid and a plasmid encoding the PAM-flanked dsDNA target upstream of a GFP reporter construct were added to a reaction. Cleavage of the reporter construct leads to loss of GFP expression through rapid degradation of the linearized DNA (Fig. 2b).

To interrogate the reprogrammability of the crRNA-tracrRNA duplex, the inventors started with the intervening bulge in the crRNA-tracrRNA duplex followed by the two stems. Previous studies showed that a bulge in the LR/AR duplex associated with BhCas12b is necessary to maintain the dsDNA targeting activity for SpyCas9 and Sth1Cas9 (49). However, removing this bulge did not impinge on GFP silencing (Fig. 2c), likely due the bulge falling outside of the nuclease binding region (51). Using the bulge-removed variant as a baseline to interrogate programmability of the LR/AR and SR/AR RNA stems (Fig. 2c), it was found that both stems could be reprogrammed without impinging on GFP silencing, whether changing the lower or upper portion of the LR/AR stem (cr1-4) or the SR/AR stem (cr5-6). The crRNA-tracrRNA duplex could be similarly reprogrammed for the *Bacillus thermoamylovorans* Cas12b (BthCas12b) (51), as changes in the LR/AR and SR/AR of a single-guide RNA (sgRNA) fusing were well tolerated. Therefore, tracrRNAs associated with Cas12b nucleases were found to be highly amenable to reprogramming.

The inventors explored the extent to which the BhCas12b Rptr could be applied for RNA detection. The inventors started with the *CJ8421_04975* mRNA previously used to evaluate Rptrs associated with different Cas9 nucleases (29). Two Rptrs hybridizing at different loci of *CJ8421_04975* mRNA were designed based on rules derived from mutational analysis of the LR/AR and SR/AR (Figs. 2c). Strong GFP silencing was observed for both BhRptr1 and BhRptr2 when compared with the non-targeting crRNA control, and both Rptrs combined with the mRNA exhibited similar performance as their equivalent crRNA/tracrRNA pairs (Fig. 2d-e). Furthermore, dsDNA targeting occurred specifically through the predicted guide sequence, as mutating the predicted seed region or scrambling the tracrRNA anti-repeat (long, short or both) fully inhibited GFP silencing. The one exception was scrambling the anti-repeat of the tracrRNA associated with locus 1 of the mRNA, which still maintained substantial targeting activity likely due to shifted base pairing in the short duplex (Fig. 2d-e). Overall, the Cas12b tracrRNA can obviously be reprogrammed to link an RNA-of-interest to sequence-specific dsDNA targeting.

### Reprogrammed tracrRNAs link RNA sensing and dsDNA targeting by Cas12e and Cas12f1 nucleases

Building on the reprogramming of Cas12b tracrRNAs, the inventors turned to the *Acidibacillus sulfuroxidans* Cas12f1 (AsCas12f1) from Type V-F CRISPR-Cas systems (37). While its crRNA-tracrRNA duplex parallels that associated with BhCas12b (Fig. 3a), AsCas12f1 is a much smaller protein and forms a homodimer when binding a single crRNA-tracrRNA duplex. Using the TXTL assay with plasmid-expressed AsCas12f1 and an sgRNA, the inventors found that the intervening bulge was also dispensable and the LR/AR and SR/AR could be fully reprogrammed without impinging on GFP silencing (Fig. 3b). The base-pairing in the SR/AR was crucial for dsDNA targeting, as deletion of the SR portion of the SR/AR or mismatches in the SR/AR substantially inhibited GFP silencing (Fig. 3b). It was further demonstrated that three Rptrs designed to hybridize to different loci in the *CJ8421_04975* mRNA yielded GFP silencing with comparable performance as their equivalent crRNA/tracrRNA counterparts in TXTL (Fig. 3c-d). As before, mutating the seed region in the predicted guide or scrambling the tracrRNA anti-repeat (long, short or both) fully inhibited GFP silencing.

Deviating from Cas12b and Cas12f1, Cas12e nucleases rely on crRNA-tracrRNA duplexes containing an RNA triple helix instead of a pseudoknot (Figs. 3e) (33, 47, 55, 56). The inventors selected the previously characterized Deltaproteobacteria Cas12e (DpbCas12e) (33) and evaluated the reprogrammability of the bulged stem as well as the triple helix. Paralleling BhCas12b and AsCas12f1, removing the bulge and a G-U wobble pair in the context of an sgRNA did not compromise GFP silencing, and the stem could be readily reprogrammed (Fig. 3f). Turning to the triple helix, this helix is formed by two separate tracts of three uracils at the 5' end of the tracrRNA sandwiching three adenosines in the repeat (Figs. 3e) (33). A *cis* Hoogsteen/Watson-Crick base pair forms between the U.A and a *cis* Watson-Crick/Watson-Crick base pair forms between the A-U, assigning the triple helix to the cWW/cHW triple family (58). RNA triple-helix motifs are found in various functional RNAs, such as telomerase RNAs (59, 60), riboswitches (61) and long noncoding RNAs (62, 63). Despite its diversified distribution, the changeability of RNA triple helix in these biologically important RNAs has not been systematically investigated.

The inventors found other RNA triple helices in the same cWW/cHW family might preserve dsDNA targeting by DpbCas12e. Using the RNA Base Triple Database as a reference (64), they tested all nine RNA triple helix combinations reported in existing functional RNAs (s11-s18, and the native U.A-U), three expected to form a triple helix but not observed to-date (s19-21), and two not expected to form a triple helix and not observed to-date (s22-23, Fig. 3g). Among the 14 tested triple-helix combinations, two (C.G-U_s11, C.G-C_s18) yielded GFP silencing comparable to that of the native U.A-U. In addition, installing the combination of C.G-U and C.G-C base triples in the RNA triple-helix region (s24-26) also yielded comparable GFP silencing. As expected, disrupting the RNA triple-helix conformation in one of three triples in the triple-helix region abolished dsDNA targeting (s27-32, Fig. 3g), indicating a stringent triple-helix conformation required by DpbCas12e.

The RNA triple-helix region is surrounded by one G-C base pair at the 3' end and three unpaired nucleotides (AUC) at the 5' end of the repeat that may also represent necessary sequence or structural features (Fig. 3h). For the G-C base pair, the inventors found that introducing a G.A mismatch (s33) fully abolished silencing, while changing the base pair to U-A (s34) only modestly reduced GFP silencing (Fig. 3h). For the AUC at the 5' end, mutating the C to A, G and U (s35-s38) resulted in similar or even improved GFP silencing (Fig. 3h). The U could also be replaced with other nucleotides (s39-s41) without compromising activity (Fig. 3h). Changing the A to C or G (s42, s44) was also well tolerated while changing the A to U (s43) substantially inhibited GFP silencing (Fig. 3h). Together, the RNA duplex and triple-helix regions are reprogrammable, albeit with less flexibility for the triple-helix region.

Based on the insights from the systematic mutational analyses to DpbCas12e sgRNA, the inventors designed three Rptrs targeting different loci in *CJ8421_04975* mRNA (Fig. 3i). They observed substantial GFP silencing for all three designed Rptrs, with comparable performance to that of their equivalent crRNA:tracrRNA pairs. As before, mutating the seed region in the predicted guide or scrambling the tracrRNA anti-repeat inhibited GFP silencing (Fig. 3j). Overall, Rptrs can be extended to different Cas12 nucleases with varying tracrRNA-crRNA structures.

### Truncating the dsDNA target enhances collateral cleavage-based RNA detection with BhCas12b and DpbCas12e

In contrast to Cas9, Cas12 non-specifically cleaves ssDNA upon target recognition, enabling signal amplification as part of CRISPR-based diagnostics (2). The inventors therefore reasoned that combining Rptrs, dsDNA targets, and ssDNA reporters would couple RNA detection by Cas12 to an amplified readable output-the basis of PUMA. To assess the collateral effects of BhCas12b, the inventors devised an *in vitro* collateral cleavage assay using purified BhCas12b protein, *in vitro*-transcribed sgRNAs or RNAs to be detected and Rptrs, linear dsDNA targets and a ssDNA fluorophore-quencher reporter (Figs. 4a). Upon recognition and cleavage of its dsDNA target, the nuclease non-specifically cleaves the fluorophore-quencher reporter, resulting in an increase in fluorescence.

The inventors began with an sgRNA and a 337-bp linear dsDNA containing a 27-bp PAM-flanked target and incubating at 37°C (Fig. 7a). They observed slight background fluorescence without the dsDNA target and a monotonically increasing fluorescence with the dsDNA target that plateaued after 12 hours (*k*_{obs} = 0.03 h⁻¹, Fig. 7b), in line with cis-cleavage of the dsDNA target triggering multi-turnover collateral cleavage of the fluorescent ssDNA reporter by BhCas12b. The activity exhibited by BhCas12b was weaker compared to FnCas12a (*k*_{obs} = 0.11 h⁻¹), DpbCas12e (*k*_{obs} = 0.65 h⁻¹) and LbCas12a (*k*_{obs} = 2.1 h⁻¹) under equivalent conditions (Fig. 7b). Elevating the temperature from 29 to 42°C gradually increased the reaction rate by 1.7-fold (*k*_{obs} = 0.19 h⁻¹ at 42°C) (Fig. 7c), in line with higher temperatures yielding optimal cleavage activity for Cas12 nucleases (10, 34, 46).

With an *in vitro* collateral cleavage assay in place, in another aspect of the present invention, the inventors turned to the dsDNA target. Standard Cas12-based diagnostics have limited control over the composition of the dsDNA target without extensive manipulations. In contrast, here, the dsDNA target is provided, granting complete control over its sequence, length, and chemistry. This control in turn can be leveraged to enhance the reaction. As a start, the inventors evaluated the impact of using targets encoded on shorter linear DNA, perceivably by reducing the search time for the target sequence. In line with this rationale, a 5.6-fold increase in collateral cleavage activity at 37°C was observed, when shortening the dsDNA target length from 337 bp (*k*_{obs} = 0.03 h⁻¹) to 94 bp (*k*_{obs} = 0.17 h⁻¹). However, collateral cleavage activity decreased when shortening the DNA length to 60 bp (*k*_{obs} = 0.12 h⁻¹) or to 48 bp (*k*_{obs} = 0.08 h⁻¹) (Fig. 7d).

The observed impact of DNA length on signal production led us to explore a distinct aspect of the dsDNA target: the extent of processing by Cas12 nucleases. Upon target recognition, Cas12 nicks the non-target stand followed by the target strand of the dsDNA target through the nuclease's RuvC domain (65, 66), leading to a cleaved dsDNA target with a 5' overhang (Fig. 4a). Complete cleavage of the dsDNA target is a prerequisite of collateral cleavage (65), with target strand cleavage posing the rate-limiting step (34, 66-68). The inventors therefore hypothesized that using a dsDNA target with a processed target strand would increase the observed rate of collateral cleavage. In line with this hypothesis, a dsDNA target with a processed non-target strand yielded similar collateral cleavage rates to that of an unprocessed dsDNA target (*k*_{obs} = 0.06 - 0.16 h⁻¹) at 37°C for dsDNA lengths ranging between 45 and 94 bp (Figs. 4b and 7d). In contrast, a dsDNA target with a processed target strand yielded increased collateral cleavage rates (*k*_{obs} up to 0.46 h⁻¹ for NTS+55 : TS+0), in line with target strand cleavage posing the rate-limiting step (Figs. 4b and 7d). A similar collateral cleavage rate (*k*_{obs} = 0.44 h⁻¹) was observed for a dsDNA target with both strands processed (NTS-6 : TS+0) (Fig. 7d). Finally, trimming the target strand by two additional nts towards the PAM can further enhance the observed collateral cleavage activity (NTS-6 : TS-2, *k_{obs}* = 0.56 h⁻¹) (Fig. 7d).

With conditions established for enhanced RNA detection using BhCas12b, the inventors turned to detecting the full-length *CJ8421_04975* mRNA using BhRptr4 *in vitro.* Under the optimal conditions with the shortest and processed dsDNA target (NTS-6:TS-2) at 42°C, the RNA to be detected was detected at 1 µM in 45 minutes and at 1 nM in 16 hours based on endpoint measurements compared to a no-RNA control (Figs. 4c and 8c).

The inventors asked whether these strategies also apply to DpbCas12e, which exhibited much higher collateral cleavage activities (Fig. 7). The inventors tested DpbCas12e with DpbRptr1 against the full-length *CJ8421_04975* mRNA along with different-sized dsDNA targets at different temperatures (Fig. 9a-c). Similar to BhCas12b, DpbCas12e exhibited increased activity when elevating the temperature (*k*_{obs} = 0.07 h⁻¹ at 29°C, 0.43 h⁻¹ at 37°C and 0.67 h⁻¹ at 42°C) (Fig. 9b) and when shortening the length of the dsDNA target (*k*_{obs} = 0.43 h⁻¹ for 334 bp and 0.54 h⁻¹ for 44 bp) (Fig. 9c). Moreover, introducing a processed dsDNA target increased the collateral cleavage rate (for 91-bp target, *k_{obs}* = 0.46 h⁻¹ for unprocessed strands, 0.78 h⁻¹ for processed target strand) (Figs. 4d and 10a). Finally, under the optimized conditions using the double-strand processed 38-bp dsDNA target at 42°C, the mRNA to be detected could be detected at a concentration of 1 µM in 9 minutes and at a concentration of 100 pM in 16 hours (Figs. 4e and 10b). Therefore, different tracrRNA-dependent Cas12 nucleases can be co-opted for direct, PAM-free RNA detection *in vitro.*

### Reprogrammed tracrRNAs circumvent background collateral activity from Cas12-gRNA complexes

When comparing collateral cleavage activities across Cas12 orthologs (Fig. 7b), the inventors noticed that the LbCas12a-sgRNA complex produces substantial fluorescence even in the absence of its corresponding dsDNA target, reaching approximately 70% of the levels seen when its dsDNA target is present after 16 hours of incubation (Fig. 7b). A high background activity was also reported for AsCas12a in previous studies (69, 70). To assess the prevalence of this background activity, the inventors tested four BhCas12b sgRNAs (#1-4, with the #2 guide used with other Cas12 orthologs in Figure 7b) using processed dsDNA targets (NTS-6 : TS-2). Substantial leaky activity was observed for sgRNA#1 and #3, with comparable fluorescence levels as those with the dsDNA targets after 16 hours (Fig. 5a). Intriguingly, sgRNA#3 exhibited similar high cleavage activity (*k*_{obs} = 1.02-1.16 h⁻¹) regardless of the presence or absence of the dsDNA target. This background activity would reduce detection sensitivity, making it more challenging to identify low-concentration substrates. In contrast, the inventors hypothesize any background activities would be greatly reduced using Rptrs, as the guide RNA is principally formed only in the presence of the RNA to be detected. Supporting this hypothesis, background collateral activity was consistently low in the presence of each Cas12b Rptr but in the absence of the corresponding RNA to be detected, with endpoint fluorescence levels 3.5-fold to 29.9-fold lower than those observed in the presence of the sgRNA (Fig. 5b). This unique feature of RNA detection with Cas12 Rptrs allows for greater sensitivity for RNA detection.

### A universal Cas12 Rptr can decipher different bacterial pathogens

One core feature of Rptrs is that base pairing with an RNA to be detected is somewhat flexible, whereas the flanking guide sequence should direct dsDNA targeting that is highly sensitive to mismatches (29). To exploit this feature, the inventors applied Cas12 Rptrs to differentiate bacterial pathogens based on their 16S rRNA (71, 72). Differentiating different pathogens can be important to select appropriate courses of treatment for different indications such as acute sepsis, urinary tract infections, or sexually transmitted diseases. Traditional CRISPR diagnostics based on collateral cleavage by Cas12 or Cas13-based diagnostics have taken strides in this direction (73), with one example using multiple guide RNAs to detect different bacterial pathogens (74). In contrast, with this core feature of Rptrs, a single Rtpr could be designed to pair next to a variable region of 16S rRNA indicating the genus. The variable region would then be matched to a dsDNA target, with its cleavage and subsequent collateral activity indicating which pathogen is present.

To determine how to best design the Rptr, the inventors began by evaluating the specificity of the three different Cas12b homologs (BthCas12b, BhCas12b and AacCas12b), with the goal of identifying at least one homolog exhibited high guide-target mismatch sensitivity. The inventors assessed collateral cleavage activity of each homolog using sgRNAs encoding the same guide sequence along with dsDNA targets containing two consecutive mismatches sliding through the guide-target region (Fig. 6a). Among the three orthologs, BthCas12b was the most sensitive to guide-target mismatches, especially in positions 5-12 proximal to the PAM, retaining only 1-11% fluorescence compared to the original dsDNA target after 1h reaction (Fig. 6a). The inventors therefore proceeded with BthCas12b and aimed for sequence differences to fall within the most sensitive positions of the target.

Following this approach, the inventors designed a single BthCas12b Rptr that hybridizes to a conserved region of bacterial 16S rRNA, with the downstream variable region serving as the guide sequence. The inventors specifically focused on five common bacterial pathogens, E. *coli, Klebsiella pneumoniae, Staphylococcus aureus, Enterococcus faecalis,* and *Listeria monocytogenes* (Fig. 6b), where the sequence differences fall within the region of sensitivity for BthCas12b. As before, a PAM did not need to appear within the RNA to be detected, as this was encoded within the dsDNA targets. The inventors then assessed collateral cleavage activity for each 16S rRNA fragment and each dsDNA target. The inventors found that the presence of the 16S rRNA fragment from one specific pathogen triggers fluorescence release only when paired with its corresponding dsDNA target (Fig. 6c). The inventors noticed 16S rRNA from *L*. *monocytogenes* also gave rise to substantial fluorescence when pairing with the dsDNA target from *S. aureus,* likely due to the high similarity between their 16S rRNA fragments with only three mismatches present outside of the seed region (Fig. 6b-c). Thus, specific detection of different pathogens based on 16S rRNA can be achieved via a single Rptr.

### Literature as cited

1. Kaminski, M. M., Abudayyeh, O. O., Gootenberg, J. S., Zhang, F. & Collins, J. J. CRISPR-based diagnostics. Nat Biomed Eng 5, 643-656 (2021).
2. Chen, J. S. et al. CRISPR-Cas12a target binding unleashes indiscriminate single-stranded DNase activity. Science 360, 436-439 (2018).
3. East-Seletsky, A. et al. Two distinct RNase activities of CRISPR-C2c2 enable guide-RNA processing and RNA detection. Nature 538, 270-273 (2016).
4. Gootenberg, J. S. et al. Nucleic acid detection with CRISPR-Cas13a/C2c2. Science 356, 438-442 (2017).
5. Makarova, K. S. et al. Evolutionary classification of CRISPR-Cas systems: a burst of class 2 and derived variants. Nat. Rev. Microbiol. 18, 67-83 (2019).
6. Urbaitis, T. et al. A new family of CRISPR-type V nucleases with C-rich PAM recognition. EMBO Rep. 23, e55481 (2022).
7. Wu, W. Y et al. The miniature CRISPR-Cas12m effector binds DNA to block transcription. Mol. Cell 82, 4487-4502.e7 (2022).
8. Chen, W. et al. Cas12n nucleases, early evolutionary intermediates of type V CRISPR, comprise a distinct family of miniature genome editors. Mol. Cell 83, 2768-2780.e6 (2023).
9. Teng, F. et al. CDetection: CRISPR-Cas12b-based DNA detection with sub-attomolar sensitivity and single-base specificity. Genome Biology vol. 20 Preprint at https://doi.org/10.1186/s13059-019-1742-z (2019).
10. Li, L. et al. HOLMESv2: A CRISPR-Cas12b-Assisted Platform for Nucleic Acid Detection and DNA Methylation Quantitation. ACS Synth. Biol. 8, 2228-2237 (2019).
11. Wang, Z. & Zhong, C. Cas12c-DETECTOR: A specific and sensitive Cas12c-based DNA detection platform. Int. J. Biol. Macromol. 193, 441-449 (2021).
12. Yan, W. X. et al. Functionally diverse type V CRISPR-Cas systems. Science 363, 88-91 (2019).
13. Dmytrenko, O. et al. Cas12a2 elicits abortive infection through RNA-triggered destruction of dsDNA. Nature 613, 588-594 (2023).
14. Bravo, J. P. K. et al. RNA targeting unleashes indiscriminate nuclease activity of CRISPR-Cas12a2. Nature 613, 582-587 (2023).
15. Li, Z., Zhang, H., Xiao, R., Han, R. & Chang, L. Cryo-EM structure of the RNA-guided ribonuclease Cas12g. Nat. Chem. Biol. 17, 387-393 (2021).
16. Wei, Y. et al. Trans single-stranded DNA cleavage via CRISPR/Cas14a1 activated by target RNA without destruction. Angew. Chem. Weinheim Bergstr. Ger. 133, 24443-24449 (2021).
17. Liang, M. et al. A CRISPR-Cas12a-derived biosensing platform for the highly sensitive detection of diverse small molecules. Nat. Commun. 10, 3672 (2019).
18. Mao, Z. et al. CRISPR/Cas12a-based technology: A powerful tool for biosensing in food safety. Trends Food Sci. Technol. 122, 211-222 (2022).
19. Zhang, D. H., Raykar, S. & Ng, K. T. C. PAM-less Exonuclease-assisted Cas12a for visual detection of Vibrio Species. bioRxiv 2022.10.21.513145 (2022) doi:10.1101/2022.10.21.513145
20. Zhou, S. et al. Endonuclease-Assisted PAM-free Recombinase Polymerase Amplification Coupling with CRISPR/Cas12a (E-PfRPA/Cas) for Sensitive Detection of DNA Methylation. ACS Sens 7, 3032-3040 (2022).
21. Ding, X. et al. Ultrasensitive and visual detection of SARS-CoV-2 using all-in-one dual CRISPR-Cas12a assay. Nat. Commun. 11, 4711 (2020).
22. Zhang, W. et al. PAM-independent ultra-specific activation of CRISPR-Cas12a via sticky-end dsDNA. Nucleic Acids Res. 50, 12674-12688 (2022).
23. Li, S.-Y et al. CRISPR-Cas12a-assisted nucleic acid detection. Cell Discov 4, 20 (2018).
24. Chen, Y, Mei, Y. & Jiang, X. Universal and high-fidelity DNA single nucleotide polymorphism detection based on a CRISPR/Cas12a biochip. Chem. Sci. 12, 4455-4462 (2021).
25. Zhu, Z. et al. PAM-free loop-mediated isothermal amplification coupled with CRISPR/Cas12a cleavage (Cas-PfLAMP) for rapid detection of rice pathogens. Biosens. Bioelectron. 204, 114076 (2022).
26. Zhong, M. et al. PCDetection: PolyA-CRISPR/Cas12a-based miRNA detection without PAM restriction. Biosens. Bioelectron. 214, 114497 (2022).
27. Rananaware, S. R. et al. Programmable RNA detection with CRISPR-Cas12a. bioRxiv (2023) doi:10.1101/2023.01.29.525716.
28. Liao, C. & Beisel, C. L. The tracrRNA in CRISPR Biology and Technologies. Annu. Rev. Genet. (2021) doi:10.1146/annurev-genet-071719-022559.
29. Jiao, C. et al. Noncanonical crRNAs derived from host transcripts enable multiplexable RNA detection by Cas9. Science 372, 941-948 (2021).
30. Jiao, C. et al. RNA recording in single bacterial cells using reprogrammed tracrRNAs. Nat. Biotechnol. (2023) doi:10.1038/s41587-022-01604-8.
31. Liu, Y. et al. Reprogrammed tracrRNAs enable repurposing of RNAs as crRNAs and sequence-specific RNA biosensors. Nat. Commun. 13, 1937 (2022).
32. Teng, F. et al. Repurposing CRISPR-Cas12b for mammalian genome engineering. Cell Discov 4, 63 (2018).
33. Liu, J.-J. et al. CasX enzymes comprise a distinct family of RNA-guided genome editors. Nature 566, 218-223 (2019).
34. Strecker, J. et al. Engineering of CRISPR-Cas12b for human genome editing. Nature Communications vol. 10 Preprint at https://doi.org/10.1038/s41467-018-08224-4 (2019).
35. Strecker, J. et al. RNA-guided DNA insertion with CRISPR-associated transposases. Science 365, 48-53 (2019).
36. Harrington, L. B. et al. A scoutRNA Is Required for Some Type V CRISPR-Cas Systems. Mol. Cell 79, 416-424.e5 (2020).
37. Wu, Z. et al. Programmed genome editing by a miniature CRISPR-Cas12f nuclease. Nat. Chem. Biol. 17, 1132-1138 (2021).
38. Bigelyte, G. et al. Miniature type VF CRISPR-Cas nucleases enable targeted DNA modification in cells. Nat. Commun. 12, 1-8 (2021).
39. Kim, D. Y. et al. Efficient CRISPR editing with a hypercompact Cas12f1 and engineered guide RNAs delivered by adeno-associated virus. Nat. Biotechnol. 40, 94-102 (2022).
40. Xu, X. et al. Engineered miniature CRISPR-Cas system for mammalian genome regulation and editing. Mol. Cell 81, 4333-4345.e4 (2021).
41. Sun, A. et al. The compact Casπ (Cas12l) 'bracelet' provides a unique structural platform for DNA manipulation. Cell Res. 33, 229-244 (2023).
42. Harrington, L. B. et al. Programmed DNA destruction by miniature CRISPR-Cas14 enzymes. Science 362, 839-842 (2018).
43. Kurihara, N. et al. Structure of the type V-C CRISPR-Cas effector enzyme. Mol. Cell (2022) doi:10.1016/j.molcel.2022.03.006.
44. Huang, C. J., Adler, B. A. & Doudna, J. A. A naturally DNase-free CRISPR-Cas12c enzyme silences gene expression. bioRxiv 2021.12.06.471469 (2021) doi: 10.1101/2021.12.06.471469.
45. Karvelis, T. et al. PAM recognition by miniature CRISPR-Cas12f nucleases triggers programmable double-stranded DNA target cleavage. Nucleic Acids Res. 48, 5016-5023 (2020).
46. Shmakov, S. et al. Discovery and Functional Characterization of Diverse Class 2 CRISPR-Cas Systems. Mol. Cell 60, 385-397 (2015).
47. Querques, I., Schmitz, M., Oberli, S., Chanez, C. & Jinek, M. Target site selection and remodelling by type V CRISPR-transposon systems. Nature 599, 497-502 (2021).
48. Deltcheva, E. et al. CRISPR RNA maturation by trans-encoded small RNA and host factor RNase III. Nature 471, 602-607 (2011).
49. Briner, A. E. et al. Guide RNA functional modules direct Cas9 activity and orthogonality. Mol. Cell 56, 333-339 (2014).
50. Dugar, G. et al. CRISPR RNA-dependent binding and cleavage of endogenous RNAs by the Campylobacter jejuni Cas9. Mol. Cell 69, 893-905.e7 (2018).
51. Wu, D., Guan, X., Zhu, Y, Ren, K. & Huang, Z. Structural basis of stringent PAM recognition by CRISPR-C2c1 in complex with sgRNA. Cell Res. 27, 705-708 (2017).
52. Liu, L. et al. C2c1-sgRNA Complex Structure Reveals RNA-Guided DNA Cleavage Mechanism. Mol. Cell 65, 310-322 (2017).
53. Xiao, R., Li, Z., Wang, S., Han, R. & Chang, L. Structural basis for substrate recognition and cleavage by the dimerization-dependent CRISPR-Cas 12f nuclease. Nucleic Acids Res. 49, 4120-4128 (2021).
54. Takeda, S. N. et al. Structure of the miniature type V-F CRISPR-Cas effector enzyme. Mol. Cell 81, 558-570.e3 (2021).
55. Tsuchida, C. A. et al. Chimeric CRISPR-CasX enzymes and guide RNAs for improved genome editing activity. Mol. Cell 82, 1199-1209.e6 (2022).
56. Xiao, R. et al. Structural basis of target DNA recognition by CRISPR-Cas12k for RNA-guided DANN transposition. Mol. Cell 81, 4457-4466.e5 (2021).
57. Marshall, R. et al. Rapid and Scalable Characterization of CRISPR Technologies Using an E. coli Cell-Free Transcription-Translation System. Mol. Cell 69, 146-157.e3 (2018).
58. Abu Almakarem, A. S., Petrov, A. I., Stombaugh, J., Zirbel, C. L. & Leontis, N. B. Comprehensive survey and geometric classification of base triples in RNA structures. Nucleic Acids Res. 40, 1407-1423 (2012).
59. Shefer, K. et al. A triple helix within a pseudoknot is a conserved and essential element of telomerase RNA. Mol. Cell. Biol. 27, 2130-2143 (2007).
60. Cash, D. D. et al. Pyrimidine motif triple helix in the Kluyveromyces lactis telomerase RNA pseudoknot is essential for function in vivo. Proc. Natl. Acad. Sci. U. S. A. 110, 10970-10975 (2013).
61. Batey, R. T. Structure and mechanism of purine-binding riboswitches. Q. Rev. Biophys. 45, 345-381 (2012).
62. Tycowski, K. T., Shu, M.-D., Borah, S., Shi, M. & Steitz, J. A. Conservation of a triple-helix-forming RNA stability element in noncoding and genomic RNAs of diverse viruses. Cell Rep. 2, 26-32 (2012).
63. Mitton-Fry, R. M., DeGregorio, S. J., Wang, J., Steitz, T. A. & Steitz, J. A. Poly(A) tail recognition by a viral RNA element through assembly of a triple helix. Science 330, 1244-1247 (2010).
64. RNA Base Triple Database. http://rna.bgsu.edu/triples/triples.php.
65. Swarts, D. C. & Jinek, M. Mechanistic Insights into the cis- and trans-Acting DNase Activities of Cas12a. Mol. Cell 73, 589-600.e4 (2019).
66. Cofsky, J. C. et al. CRISPR-Cas12a exploits R-loop asymmetry to form double-strand breaks. Elife 9, (2020).
67. Jeon, Y. et al. Direct observation of DNA target searching and cleavage by CRISPR-Cas12a. Nat. Commun. 9, 2777 (2018).
68. Strohkendl, I., Saifuddin, F. A., Rybarski, J. R., Finkelstein, I. J. & Russell, R. Kinetic Basis for DNA Target Specificity of CRISPR-Cas12a. Mol. Cell 71, 816-824.e3 (2018).
69. Liang, Y. et al. CRISPR-Cas12a-Based Detection for the Major SARS-CoV-2 Variants of Concern. Microbiol Spectr 9, e0101721 (2021).
70. Ooi, K. H. et al. An engineered CRISPR-Cas12a variant and DNA-RNA hybrid guides enable robust and rapid COVID-19 testing. Nat. Commun. 12, 1739 (2021).
71. Weisburg, W. G., Barns, S. M., Pelletier, D. A. & Lane, D. J. 16S ribosomal DNA amplification for phylogenetic study. J. Bacteriol. 173, 697-703 (1991).
72. Srinivasan, R. et al. Use of 16S rRNA gene for identification of a broad range of clinically relevant bacterial pathogens. PLoS One 10, e0117617 (2015).
73. CRISPR-Cas-based techniques for pathogen detection: Retrospect, recent advances, and future perspectives. Journal of Advanced Research 50, 69-82 (2023).
74. Thakku, S. G. et al. Multiplexed detection of bacterial nucleic acids using Cas13 in droplet microarrays. PNAS Nexus 1, gac021 (2022).
75. Huyke, D. A. et al. Enzyme Kinetics and Detector Sensitivity Determine Limits of Detection of Amplification-Free CRISPR-Cas12 and CRISPR-Cas13 Diagnostics. Anal. Chem. 94, 9826-9834 (2022).
76. Ramachandran, A. et al. Electric field-driven microfluidics for rapid CRISPR-based diagnostics and its application to detection of SARS-CoV-2. Proceedings of the National Academy of Sciences 117, 29518-29525 (2020).
77. Schmitz, M., Querques, I., Oberli, S., Chanez, C. & Jinek, M. Structural basis for the assembly of the type V CRISPR-associated transposon complex. Cell 185, 4999-5010.e17 (2022).
78. Zhang, B. et al. Structural insights into target DNA recognition and cleavage by the CRISPR-Cas12c1 system. Nucleic Acids Res. 50, 11820-11833 (2022).
79. Wu, D., Guan, X., Zhu, Y. & Huang, Z. Crystal structure of the CRISPR-associated protein in complex with crRNA and DNA. Preprint at https://doi.org/10.2210/pdb5wti/pdb (2017).
80. Ming, M. et al. CRISPR-Cas 12b enables efficient plant genome engineering. Nature Plants 6, 202-208 (2020).
81. Burstein, D. et al. New CRISPR-Cas systems from uncultivated microbes. Nature 542, 237-241 (2016).
82. Shin, J. & Noireaux, V. Efficient cell-free expression with the endogenous E. Coli RNA polymerase and sigma factor 70. J. Biol. Eng. 4, 8 (2010).

## Claims

1. A CRISPR diagnostic method for detecting at least one specific dsDNA target nucleic acid, comprising the steps of binding the at least one dsDNA target nucleic acid by a Cas nuclease enzyme, wherein the Cas nuclease enzyme is selected from a Cas enzyme binding to dsDNA nucleic acid molecules, such as, for example, Cas9 or Cas12, in particular Cas12a, Cas12b, Cas12c, Cas12d, Cas12e, Cas12f1, Cas12g, Cas12i, and Cas12l, and
subsequently cleaving of at least one marker nucleic acid by collateral nuclease activity of the Cas nuclease enzyme,
comprising providing the at least one dsDNA target nucleic acid comprising a target strand (TS) sequence comprising a Cas nuclease cleavage site and a non-target strand (NTS) sequence forming a TS:NTS duplex, a protospacer adjacent motif (PAM) sequence directly located upstream or downstream of the TS:NTS duplex, and a sequence extending upstream and/or downstream from the PAM and/or TS:NTS duplex,
wherein before the step of binding the dsDNA target nucleic acid molecule by the Cas nuclease enzyme, the sequence extending from the TS:NTS duplex is shortened to 30 base pairs or less, and is preferably eliminated and if the downstream extended sequence is eliminated, the TS sequence is shortened between 0 to 8, preferably between 2 to 4, nucleotide bases and/or the NTS sequence is truncated between 0 to 10, preferably between 6 to 8, nucleotide bases.

2. The method according to claim 1, wherein the length of the shortened at least one dsDNA target nucleic acid is between about 120 and about 24 bp, preferably between about 100 and about 30 bp, more preferably at between about 60 and about 35 bp.

3. The method according to claim 1 or 2, wherein the shortening the dsDNA target nucleic acid molecule comprises the steps of introducing a cleavage site into the dsDNA target nucleic acid molecule and subsequent enzymatic cleavage, preferably using DNA amplification with primers that are modified to include the cleavage site, or wherein the dsDNA target nucleic acid molecule is provided as a synthesized shortened molecule, wherein preferably cleaving comprises cleaving with an enzyme selected from restriction enzymes, Cas nucleases, meganucleases, zinc-finger nucleases, and TALE nucleases, and wherein preferably the cleavage site is a restriction site that is preferably recognized by an IIS restriction enzyme, such as, for example, Acul, Bbyl, or NmeAIII.

4. The method according to any one of claims 1 to 3, wherein the shortened at least one dsDNA target nucleic acid is arranged as 5'-upstream extending sequence - PAM - target sequence - downstream extending sequence-3', or as 5'-upstream extending sequence - PAM - target sequence-3', or 5'-upstream extending sequence - PAM - shortened target sequence-3'.

5. The method according to any one of claims 1 to 4, wherein the TS sequence has a length of between about 20 to 40 nt, preferably between about 20 to 30 nt, more preferred about 24 nt.

6. The method according to any one of claims 1 to 5, wherein the at least one marker nucleic acid is selected from ssDNA, RNA, and mixtures or derivatives thereof, and/or wherein cleaving the at least one marker nucleic acid is detected, for example comprising a fluorescence marker.

7. The method according to any one of claims 1 to 6, wherein the targeting of the at least one dsDNA target nucleic acid by a Cas nuclease enzyme is dependent on an RNA molecule to be detected, preferably an mRNA molecule to be detected.

8. The method according to any one of claims 1 to 7, wherein the method is performed in the absence of gRNA molecules.

9. Use of a dsDNA target nucleic acid molecule for the CRISPR diagnostic method according to any one of claims 1 to 8, wherein the dsDNA target nucleic acid molecule comprises a target strand (TS) sequence comprising a Cas nuclease cleavage site and an opposite non-target strand (NTS) sequence, a protospacer adjacent motif (PAM) sequence directly located upstream or downstream the TS:NTS duplex, and a sequence extending upstream and/or downstream from the PAM and/or TS:NTS duplex, wherein the sequence extending downstream has a length of 30 base pairs or less, and is preferably missing and if the downstream extended sequence is missing, the TS sequence is truncated for between 0 to 8, preferably between 2 to 4, nucleotide bases and/or the NTS sequence is truncated for between 0 to 10, preferably between 6 to 8, nucleotide bases, wherein preferably the total length of the at least one dsDNA target nucleic acid is between about 120 and about 24 bp, preferably between about 100 and about 30 bp, more preferably at between about 60 and about 35 bp, wherein preferably the PAM sequence is located directly upstream and/or or directly downstream from the TS sequence.

10. The use of the dsDNA target nucleic acid molecule for a CRISPR diagnostic method according to claim 9, wherein the dsDNA target nucleic acid molecule is cleaved by an enzyme having cleavage activity, such as an IIS restriction enzyme, such as, for example, Acul, Bbyl, or NmeAIII.

11. The use of the dsDNA target nucleic acid molecule for a CRISPR diagnostic method according to claim 9 or 10, wherein the dsDNA target nucleic acid is arranged as 5'-upstream extending sequence - PAM - target sequence - downstream extending sequence-3', or as 5'-upstream extending sequence - PAM - target sequence-3', or 5'-upstream extending sequence - PAM - shortened target sequence-3'.

12. The use of the dsDNA target nucleic acid molecule according to any one of claims 9 to 11 in a CRISPR diagnostic method for detecting a specific RNA molecule, preferably an mRNA molecule, a non-coding RNA, a viral RNA molecule, or an in vitro-transcribed RNA from a DNA to be detected, wherein preferably the method is performed in vitro, and optionally the at least one specific RNA molecule is detected in a sample.

13. Use of a kit for performing the method according to any one of claims 1 to 8, comprising at least one dsDNA target nucleic acid molecule comprising a target strand (TS) sequence comprising a Cas nuclease cleavage site and an opposite non-target strand (NTS) sequence, a protospacer adjacent motif (PAM) sequence directly located upstream or downstream the TS:NTS duplex, and a sequence extending upstream and/or downstream from the PAM and/or TS:NTS duplex, wherein the sequence extending downstream has a length of 30 base pairs or less, and is preferably missing and if the downstream extended sequence is missing, the TS sequence is truncated for between 0 to 8, preferably between 2 to 4, nucleotide bases and/or the NTS sequence is truncated for between 0 to 10, preferably between 6 to 8, nucleotide bases, wherein preferably the total length of the at least one dsDNA target nucleic acid is between about 120 and about 24 bp, preferably between about 100 and about 30 bp, more preferably at between about 60 and about 35 bp, wherein preferably the PAM sequence is located directly upstream and/or or directly downstream from the TS sequence together with suitable auxiliary agents, and optionally at least one of i) a Cas enzyme binding to dsDNA nucleic acid molecules, ii) a restriction enzyme, such as an IIS restriction enzyme, such as, for example, Acul, Bbyl, or NmeAIII, iii) at least one marker nucleic acid comprising a suitable marker, for example comprising a fluorescence marker, and iv) instructions for use, preferably comprising suitable materials for a lateral flow assay.

14. Use of the kit according to claim 13 for detecting a specific RNA molecule, preferably an mRNA molecule, a non-coding RNA, a viral RNA molecule, or an in vitro-transcribed RNA from a DNA to be detected in a CRISPR diagnostic method.

## Patentansprüche

1. CRISPR diagnostisches Verfahren zum Nachweis von mindestens einer spezifischen dsDNA Ziel-Nukleinsäure,
umfassend die Schritte von Binden der mindestens einen dsDNA Ziel-Nukleinsäure durch ein Cas Nukleaseenzym, wobei das Cas Nukleaseenzym ausgewählt ist aus einem Cas Enzym, das an dsDNA Nukleinsäuremoleküle bindet, wie zum Beispiel Cas9 oder Cas12, insbesondere Cas12a, Cas12b, Cas12c, Cas12d, Cas12e, Cas12f1, Cas12g, Cas12i und Cas121 und
anschließendem Spalten der mindestens einen Marker-Nukleinsäure durch die kollaterale Nukleaseaktivität des Cas Nukleaseenzyms,
umfassend zur Verfügung stellen der mindestens einen dsDNA Ziel-Nukleinsäure umfassend eine Zielstrang (TS)-Sequenz umfassend eine Cas-Nuklease Spaltstelle und eine nicht-Zielstrang (NTS)-Sequenz, die eine TS:NTS Duplex bilden, eine Protospacer-angrenzendes Motiv (PAM) Sequenz lokalisiert direkt stromaufwärts oder stromabwärts der TS:NTS Duplex, und eine Sequenz die sich stromaufwärts und/oder stromabwärts von der PAM und/oder TS:NTS Duplex erstreckt,
wobei vor dem Schritt von Bindung des dsDNA Ziel-Nukleinsäuremoleküls durch das Cas Nukleaseenzym, die Sequenz die sich von der TS:NTS Duplex erstreckt auf 30 Basenpaare oder weniger gekürzt wird,
und bevorzugt eliminiert wird und wenn die sich stromabwärts erstreckende Sequenz eliminiert wird, die TS Sequenz zwischen 0 bis 8, bevorzugt zwischen 2 bis 4, Nukleotidbasen gekürzt wird und/oder die NTS Sequenz zwischen 0 bis 10, bevorzugt zwischen 6 bis 8, Nukleotidbasen gekürzt wird.

2. Verfahren nach Anspruch 1, wobei die Länge der verkürzten mindestens einen dsDNA Ziel-Nukleinsäure zwischen ungefähr 120 und ungefähr 24 bp, bevorzugt zwischen ungefähr 100 und ungefähr 30 bp, weiter bevorzugt zwischen ungefähr 60 und ungefähr 35 bp ist.

3. Verfahren nach Anspruch 1 oder 2, wobei die Verkürzung des dsDNA Ziel-Nukleinsäuremoleküls die Schritte von einführen einer Spaltstelle in das dsDNA Ziel-Nukleinsäuremolekül und anschließende enzymatische Spaltung umfasst, bevorzugt unter Verwendung von DNA Amplifikation mit Primern die modifiziert sind, um die Spaltstelle einzuschließen, oder wobei das dsDNA Ziel-Nukleinsäuremolekül als ein synthetisiertes verkürztes Molekül zur Verfügung gestellt wird, wobei bevorzugt das Spalten ein Spalten mit einem Enzym ausgewählt aus Restriktionsenzymen, Cas-Nukleasen, Meganukleasen, Zink-Finger-Nukleasen und TALE Nukleasen umfasst, und wobei bevorzugt die Spaltstelle eine Restriktionsstelle ist, die bevorzugt durch ein IIS Restriktionsenzym, wie zum Beispiel AcuI, BbyI, oder NmeAIII, erkannt wird.

4. Verfahren nach einem der Ansprüche 1 bis 3, wobei die verkürzte mindestens eine dsDNA Ziel-Nukleinsäure als 5'-stromaufwärts erstreckende Sequenz - PAM - Zielsequenz - stromabwärts erstreckende Sequenz-3' oder als 5'-stromaufwärts erstreckende Sequenz - PAM - Zielsequenz -3' oder 5'-stromaufwärts erstreckende Sequenz - PAM - verkürzte Zielsequenz-3' angeordnet ist.

5. Verfahren nach einem der Ansprüche 1 bis 4, wobei die TS Sequenz eine Länge von zwischen ungefähr 20 bis 40 nt, bevorzugt zwischen ungefähr 20 bis 30 nt, weiter bevorzugt ungefähr 24 nt, aufweist.

6. Verfahren nach einem der Ansprüche 1 bis 5, wobei die mindestens eine MarkerNukleinsäure ausgewählt ist aus ssDNA, RNA und Gemischen oder Derivaten davon und/oder wobei das Spalten der mindestens einen Marker-Nukleinsäure nachgewiesen wird, zum Beispiel umfassend einen Fluoreszenzmarker.

7. Verfahren nach einem der Ansprüche 1 bis 6, wobei das Abzielen der mindestens einen dsDNA Ziel-Nukleinsäure durch ein Cas Nukleaseenzym von einem nachzuweisenden RNA Molekül abhängig ist, bevorzugt einem nachzuweisenden mRNA Molekül.

8. Verfahren nach einem der Ansprüche 1 bis 7, wobei das Verfahren in der Abwesenheit von gRNA Molekülen durchgeführt wird.

9. Verwendung eines dsDNA Ziel-Nukleinsäuremoleküls für das CRISPR diagnostische Verfahren nach einem der Ansprüche 1 bis 8, wobei das dsDNA Ziel-Nukleinsäuremolekül eine Zielstrang-(TS) Sequenz eine Cas Nuklease Spaltstelle und eine gegenüberliegende nicht-Zielstrang-(NTS) Sequenz, eine Protospacer angrenzendes Motiv (PAM) Sequenz direkt stromaufwärts oder stromabwärts der TS:NTS Duplex lokalisiert umfasst, und eine Sequenz, die sich stromaufwärts und/oder stromabwärts von der PAM und/oder der TS:NTS Duplex erstreckt umfasst, wobei die sich stromabwärts erstreckende Sequenz eine Länge von 30 Basenpaaren oder weniger aufweist, und bevorzugt fehlt, und wenn die sich stromabwärts erstreckende Sequenz fehlt, die TS Sequenz zwischen 0 bis 8, bevorzugt zwischen 2 bis 4, Nukleotidbasen verkürzt ist und/oder die NTS Sequenz zwischen 0 bis 10, bevorzugt zwischen 6 bis 8, Nukleotidbasen verkürzt ist, wobei bevorzugt die Gesamtlänge der mindestens einen dsDNA Ziel-Nukleinsäure zwischen ungefähr 120 und ungefähr 24 bp, bevorzugt zwischen ungefähr 100 und ungefähr 30 bp, weiter bevorzugt bei zwischen ungefähr 60 und ungefähr 35 bp, wobei bevorzugt die PAM Sequenz direkt stromaufwärts und/oder direkt stromabwärts von der TS Sequenz lokalisiert ist.

10. Verwendung des dsDNA Ziel-Nukleinsäuremoleküls für ein CRISPR diagnostisches Verfahren nach Anspruch 9, wobei das dsDNA Ziel-Nukleinsäuremolekül durch ein Enzym mit Spaltaktivität, wie zum Beispiel einem IIS Restriktionsenzym, wie zum Beispiel AcuI, BbyI, oder NmeAIII, gespalten wird.

11. Verwendung des dsDNA Ziel-Nukleinsäuremoleküls für ein CRISPR diagnostisches Verfahren nach Anspruch 9 oder 10, wobei die dsDNA Ziel-Nukleinsäure als 5'-stromaufwärts erstreckende Sequenz - PAM - Zielsequenz - stromabwärts erstreckende Sequenz-3' oder als 5'-stromaufwärts erstreckende Sequenz - PAM - Zielsequenz -3' oder 5'-stromaufwärts erstreckende Sequenz - PAM - verkürzte Zielsequenz-3' angeordnet ist.

12. Verwendung des dsDNA Ziel-Nukleinsäuremoleküls nach einem der Ansprüche 9 bis 11 in einem CRISPR diagnostischen Verfahren zum Nachweis eines spezifischen RNA Moleküls, bevorzugt eines mRNA Moleküls, einer nicht-kodierenden RNA, einem viralen RNA Molekül oder einer in vitro-transkribierten RNA von einer nachzuweisenden DNA, wobei bevorzugt das Verfahren in vitro durchgeführt wird und gegebenenfalls das mindestens eine spezifische RNA Molekül in einer Probe nachgewiesen wird.

13. Verwendung eines Kits zur Durchführung des Verfahrens nach einem der Ansprüche 1 bis 8, umfassend mindestens ein dsDNA Ziel-Nukleinsäuremolekül umfassend eine Zielstrang-(TS) Sequenz umfassend eine Cas Nuklease Spaltstelle und eine gegenüberliegende nicht-Zielstrang- (NTS) Sequenz, eine Protospacer angrenzendes Motiv (PAM) Sequenz direkt stromaufwärts oder stromabwärts der TS:NTS Duplex lokalisiert und eine Sequenz, die sich stromaufwärts und/oder stromabwärts von der PAM und/oder TS:NTS Duplex erstreckt, wobei die Sequenz die sich stromabwärts erstreckt eine Länge von 30 Basenpaaren der weniger aufweist und bevorzugt fehlt und wenn die sich stromabwärts erstreckende Sequenz fehlt die TS Sequenz für zwischen 0 bis 8, bevorzugt zwischen 2 bis 4, Nukleotidbasen verkürzt ist und/oder die NTS Sequenz für zwischen 0 bis 10, bevorzugt zwischen 6 bis 8, Nukleotidbasen verkürzt ist, wobei bevorzugt die Gesamtlänge der mindestens einen dsDNA Ziel-Nukleinsäure zwischen ungefähr 120 und ungefähr 24 bp, bevorzugt zwischen ungefähr 100 und ungefähr 30 bp, weiter bevorzugt bei zwischen ungefähr 60 und ungefähr 35 bp ist, wobei bevorzugt die PAM Sequenz direkt stromaufwärts und/oder direkt stromabwärts von der TS Sequenz lokalisiert ist, zusammen mit geeignete Hilfsstoffen, und gegebenenfalls mindestens Einem von i) einem Cas Enzym, das an dsDNA Nukleinsäuremoleküle bindet, ii) einem Restriktionsenzym, wie zum Beispiel einem IIS Restriktionsenzym, wie zum Beispiel AcuI, BbyI oder NmeAIII, iii) mindestens einer Markernukleinsäure umfassend einen geeigneten Marker, zum Beispiel umfassend einen Fluoreszenzmarker und iv) Gebrauchsanweisungen, bevorzugt umfassend geeignete Materialen für einen Lateral Flow Assay.

14. Verwendung des Kits nach Anspruch 13 zum Nachweisen eines spezifischen RNA Moleküls, bevorzugt eines mRNA Moleküls, einer nicht-kodierenden RNA, eines viralen RNA Moleküls oder einer in vitro-transkribierten RNA von einer in einem CRISPR diagnostischen Verfahren nachzuweisenden DNA.

## Revendications

1. Procédé de diagnostic CRISPR permettant de détecter au moins un acide nucléique cible d'ADNdb spécifique, comprenant les étapes consistant à lier l'au moins un acide nucléique cible d'ADNdb par une enzyme nucléase Cas, dans lequel l'enzyme nucléase Cas est choisie parmi une enzyme Cas se liant à des molécules d'acide nucléique d'ADNdb, telle que, par exemple, Cas9 ou Cas12, en particulier Cas12a, Cas12b, Cas12c, Cas12d, Cas12e, Cas12f1, Cas12g, Cas12i, et Cas12l, et
à cliver ultérieurement au moins un acide nucléique marqueur par activité nucléasique collatérale de l'enzyme nucléase Cas,
comprenant la fourniture de l'au moins un acide nucléique cible d'ADNdb comprenant une séquence de brin cible (TS) comprenant un site de clivage de nucléase Cas et une séquence de brin non cible (NTS) formant un duplex TS:NTS, une séquence de motif adjacent à un protoespaceur (PAM) localisée directement en amont ou en aval du duplex TS:NTS, et une séquence d'extension en amont et/ou en aval du PAM et/ou du duplex TS:NTS,
dans lequel avant l'étape de liaison de la molécule d'acide nucléique cible d'ADNdb par l'enzyme nucléase Cas, la séquence s'étendant à partir du duplex TS:NTS est raccourcie à 30 paires de base ou moins, et est de préférence éliminée et si la séquence s'étendant en aval est éliminée, la séquence TS est raccourcie entre 0 à 8, de préférence entre 2 à 4, bases nucléotidiques et/ou la séquence NTS est tronquée entre 0 à 10, de préférence entre 6 à 8, bases nucléotidiques.

2. Procédé selon la revendication 1, dans lequel la longueur de l'au moins un acide nucléique cible d'ADNdb raccourci est comprise entre environ 120 et environ 24 pb, de préférence entre environ 100 et environ 30 pb, plus préférablement entre environ 60 et environ 35 pb.

3. Procédé selon la revendication 1 ou 2, dans lequel le raccourcissement de la molécule d'acide nucléique cible d'ADNdb comprend l'étape d'introduction d'un site de clivage dans la molécule d'acide nucléique cible d'ADNdb et le clivage enzymatique ultérieur, de préférence à l'aide d'une amplification d'ADN avec des amorces qui sont modifiées pour comporter le site de clivage, ou dans lequel la molécule d'acide nucléique cible d'ADNdb est fournie en tant que molécule raccourcie synthétisée, dans lequel de préférence le clivage comprend le clivage avec une enzyme choisie parmi enzymes de restriction, nucléases Cas, méganucléases, nucléases à doigt de zinc et nucléases TALE, et dans lequel de préférence le site de clivage est un site de restriction qui est de préférence reconnu par une enzyme de restriction IIS, telle que, par exemple, Acul, Bbyl ou NmeAIII.

4. Procédé selon l'une quelconque des revendications 1 à 3, dans lequel l'au moins un acide nucléique cible d'ADNdb raccourci est agencé en tant que séquence s'étendant en amont 5' - PAM - séquence cible - séquence s'étendant en aval 3', ou séquence s'étendant en amont 5' - PAM - séquence cible 3', ou séquence s'étendant en amont 5' - PAM - séquence cible raccourcie 3'.

5. Procédé selon l'une quelconque des revendications 1 à 4, dans lequel la séquence TS a une longueur comprise d'environ 20 à 40 nt, de préférence comprise d'environ 20 à 30 nt, plus préférablement d'environ 24 nt.

6. Procédé selon l'une quelconque des revendications 1 à 5, dans lequel l'au moins un acide nucléique marqueur est choisi parmi ADNsb, ARN, et mélanges ou dérivés de ceux-ci, et/ou dans lequel le clivage de l'au moins un acide nucléique marqueur est détecté, comprenant par exemple un marqueur de fluorescence.

7. Procédé selon l'une quelconque des revendications 1 à 6, dans lequel le ciblage de l'au moins un acide nucléique cible d'ADNdb par une enzyme nucléase Cas est dépendant d'une molécule d'ARN à détecter, de préférence d'une molécule d'ARNm à détecter.

8. Procédé selon l'une quelconque des revendications 1 à 7, dans lequel le procédé est mis en œuvre en l'absence de molécules d'ARNg.

9. Utilisation d'une molécule d'acide nucléique cible d'ADNdb pour le procédé de diagnostic CRISPR selon l'une quelconque des revendications 1 à 8, dans laquelle la molécule d'acide nucléique cible d'ADNdb comprend une séquence de brin cible (TS) comprenant un site de clivage de nucléase Cas et une séquence de brin non cible (NTS) opposée, une séquence de motif adjacent à un protoespaceur (PAM) localisée directement en amont ou en aval du duplex TS:NTS, et une séquence s'étendant en amont et/ou en aval du PAM et/ou du duplex TS:NTS, dans laquelle la séquence s'étendant en aval a une longueur de 30 paires de base ou moins, et est de préférence manquante et si la séquence s'étendant en aval est manquante, la séquence TS est tronquée pour entre 0 à 8, de préférence entre 2 à 4, bases nucléotidiques et/ou la séquence NTS est tronquée pour entre 0 à 10, de préférence entre 6 à 8, bases nucléotidiques, dans laquelle de préférence la longueur totale de l'au moins un acide nucléique cible d'ADNdb est comprise entre environ 120 et environ 24 pb, de préférence entre environ 100 et environ 30 pb, plus préférablement entre environ 60 et environ 35 pb, dans laquelle de préférence la séquence PAM est localisée directement en amont et/ou directement en aval de la séquence TS.

10. Utilisation de la molécule d'acide nucléique cible d'ADNdb pour un procédé de diagnostic CRISPR selon la revendication 9, dans laquelle la molécule d'acide nucléique cible d'ADNdb est clivée par une enzyme ayant une activité de clivage, telle qu'une enzyme de restriction IIS, telle que, par exemple, Acul, Bbyl ou NmeAIII.

11. Utilisation de la molécule d'acide nucléique cible d'ADNdb pour un procédé de diagnostic CRISPR selon la revendication 9 ou 10, dans laquelle l'acide nucléique cible d'ADNdb est agencé en tant que séquence s'étendant en amont 5' - PAM - séquence cible - séquence s'étendant en aval 3', ou séquence s'étendant en amont 5' - PAM - séquence cible 3', ou séquence s'étendant en amont 5' - PAM - séquence cible raccourcie 3'.

12. Utilisation de la molécule d'acide nucléique cible d'ADNdb selon l'une quelconque des revendications 9 à 11 dans un procédé de diagnostic CRISPR permettant de détecter une molécule d'ARN spécifique, de préférence une molécule d'ARNm, un ARN non codant, une molécule d'ARN viral ou un ARN transcrit in vitro à partir d'un ADN à détecter, dans laquelle de préférence le procédé est mis en œuvre in vitro, et facultativement l'au moins une molécule d'ARN spécifique est détectée dans un échantillon.

13. Utilisation d'une trousse permettant de mettre en œuvre le procédé selon l'une quelconque des revendications 1 à 8, comprenant au moins une molécule d'acide nucléique cible d'ADNdb comprenant une séquence de brin cible (TS) comprenant un site de clivage de nucléase Cas et une séquence de brin non cible (NTS) opposée, une séquence de motif adjacent à un protoespaceur (PAM) localisée directement en amont ou en aval du duplex TS:NTS, et une séquence s'étendant en amont et/ou en aval du PAM et/ou du duplex TS:NTS, dans laquelle la séquence s'étendant en aval a une longueur de 30 paires de base ou moins, et est de préférence manquante et si la séquence s'étendant en aval est manquante, la séquence TS est tronquée pour entre 0 à 8, de préférence entre 2 à 4, bases nucléotidiques et/ou la séquence NTS est tronquée pour entre 0 à 10, de préférence entre 6 à 8, bases nucléotidiques, dans laquelle de préférence la longueur totale de l'au moins un acide nucléique cible d'ADNdb est comprise entre environ 120 et environ 24 pb, de préférence entre environ 100 et environ 30 pb, plus préférablement entre environ 60 et environ 35 pb, dans laquelle de préférence la séquence PAM est localisée directement en amont et/ou directement en aval de la séquence TS conjointement avec des agents auxiliaires appropriés, et facultativement au moins l'un parmi i) une enzyme Cas se liant à des molécules d'acide nucléique d'ADNdb, ii) une enzyme de restriction, telle qu'une enzyme de restriction IIS, telle que, par exemple, Acul, Bbyl ou NmeAIII, iii) au moins un acide nucléique marqueur comprenant un marqueur approprié, comprenant par exemple un marqueur de fluorescence, et iv) des instructions d'utilisation, comprenant de préférence des matériaux appropriés pour un dosage à écoulement latéral.

14. Utilisation de la trousse selon la revendication 13 pour la détection d'une molécule d'ARN spécifique, de préférence une molécule d'ARNm, un ARN non codant, une molécule d'ARN viral ou un ARN transcrit in vitro à partir d'un ADN à détecter dans un procédé de diagnostic CRISPR.
